(19) Europäisches Patentamt
European Patent Office
Office européen des brevets

(11) Veröffentlichungsnummer: **0 017 727 A1**

# (12) EUROPÄISCHE PATENTANMELDUNG

(21) Anmeldenummer: **80100821.0**

(22) Anmeldetag: **19.02.80**

(51) Int. Cl.[3]: **C 07 D 471/04**, A 61 K 31/435 // (C07D471/04, 221/00, 209/00)

(30) Priorität: **10.04.79 CH 3408/79**

(43) Veröffentlichungstag der Anmeldung: **29.10.80 Patentblatt 80/22**

(84) Benannte Vertragsstaaten: **AT BE CH DE FR GB IT LU NL SE**

(71) Anmelder: **CIBA-GEIGY AG, Patentabteilung Postfach, CH-4002 Basel (CH)**

(72) Erfinder: **Storni, Angelo, Dr., Im Feuerbusch 3, CH-4310 Rheinfelden (CH)**
Erfinder: **Züst, Armin, Dr., Rüttihardstrasse 3, CH-4127 Birsfelden (CH)**
Erfinder: **Paioni, Romeo, Dr., Thiersteinerstrasse 22, CH-4153 Reinach (CH)**

(74) Vertreter: **Zumstein sen., Fritz, Dr. et al, Bräuhausstrasse 4, D-8000 München 2 (DE)**

## (54) Polysubstituierte Diazatricyclen, Verfahren zu ihrer Herstellung und sie enthaltende pharmazeutische Zusammensetzungen.

(57) Die vorliegende Erfindung betrifft neue Diazatricyclen der Formel

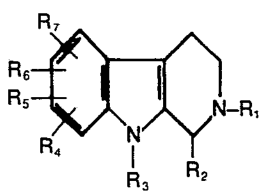

(I)

worin

$R_1$ Wasserstoff, Niederalkyl, Niederalkenyl, 2-Propinyl, Cycloalkylniederalkyl, Hydroxyniederalkyl oder gegebenenfalls substituiertes Benzyl,

$R_2$ Wasserstoff oder Niederalkyl,

$R_3$ Wasserstoff oder Niederalkyl bedeutet,

$R_4$ und $R_5$ Niederalkyl, und

$R_6$ und $R_7$ Wasserstoff, Niederalkyl, Niederalkoxy oder Halogen darstellen,

sowie Salze von solchen Verbindungen, Verfahren zur Herstellung dieser Verbindungen und ihrer Salze, solche enthaltende pharmazeutische Präparate und ihre Verwendung als Antidepressiva.

EP 0 017 727 A1

CIBA-GEIGY AG
Basel (Schweiz)

4-12300/+

**BEZEICHNUNG GEÄNDERT
siehe Titelseite**

## Polysubstituierte Diazatricyclen

Die Erfindung betrifft neue Diazatricyclen, ferner Verfahren zu deren Herstellung, sowie pharmazeutische Präparate, die diese neuen Verbindungen enthalten, wie auch ihre Verwendung.

Die Erfindung betrifft insbesondere neue Diazatricyclen der Formel

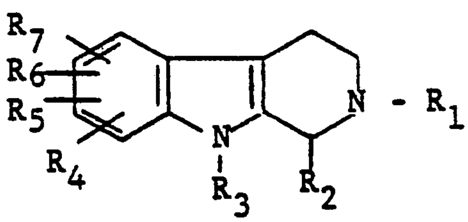

(I)

worin

$R_1$ Wasserstoff, Niederalkyl, Niederalkenyl, 2-Propinyl, Cycloalkylniederalkyl, Hydroxyniederalkyl oder gegebenenfalls substituiertes Benzyl,

$R_2$ Wasserstoff oder Niederalkyl,

$R_3$ Wasserstoff oder Niederalkyl bedeutet,

$R_4$ und $R_5$ Niederalkyl, und

$R_6$ und $R_7$ Wasserstoff, Niederalkyl, Niederalkoxy oder Halogen darstellen,

sowie Salze von solchen Verbindungen.

Die im Zusammenhang mit "nieder" bezeichneten Reste und Verbindungen enthalten vorzugsweise bis 7 und in erster Linie bis 4 Kohlenstoffatome.

Niederalkyl ist z.B. Methyl, Aethyl, n-Propyl, Isopropyl, n-Butyl, Isobutyl, sek.-Butyl oder tert.-Butyl, ferner n-Pentyl, Isopentyl, n-Hexyl, Isohexyl oder n-Heptyl.

Niederalkenyl ist z.B. Vinyl, Allyl, 2-Methylallyl, 2-Butenyl oder 3,3-Dimethylallyl.

Cycloalkylniederalkyl hat vorzugsweise 4-10, insbesondere 4-8 Kohlenstoffatome und ist z.B. Cyclopropylmethyl, Cyclopentylmethyl, Cyclohexylmethyl, 2-Cyclohexyläthyl oder Cycloheptylmethyl.

Hydroxyniederalkyl in welchem die Hydroxygruppe durch mindestens 2 C-Atome vom Stickstoff getrennt ist, ist z.B. 2-Hydroxyäthyl, ferner 2- oder 3-Hydroxypropyl.

Niederalkoxy ist z.B. Methoxy, Aethoxy, n-Propyloxy, Isopropyloxy oder n-Butyloxy.

Halogen steht für Fluor oder Brom, vorzugsweise jedoch Chlor.

Die neuen Verbindungen können in Form ihrer Salze, wie ihrer Säureadditionssalze und in erster Linie ihrer pharmazeutisch verwenbaren, nicht toxischen Säureadditionssalze vorliegen. Geeignete Salze sind z.B. solche mit anorganischen Säuren, wie Halogenwasserstoffsäuren, z.B. Chlorwasserstoffsäure oder Bromwasserstoffsäure, Schwefelsäuren, z.B. Schwefelsäure, oder Phosphorsäuren, oder mit organischen Säuren, wie aliphatischen, cycloaliphatischen, aromatischen oder heterocyclischen Carbon- oder Sulfonsäuren, z.B. Ameisen-, Essig-, Propion-, Bernstein-, Glykol-, Milch-, Aepfel-, Wein-, Zitronen-, Malein-, Hydroxymalein-, Brenztrauben-, Fumar-, Benzoe-, 4-Aminobenzoe-, Anthranil-, 4-Hydroxybenzoe-, Salicyl-, 4-Aminosalicyl-, Embon-, Methansulfon-, Aethansulfon-, 2-Hydroxyäthansulfon-, Aethylensulfon-, Toluolsulfon-, Naphthalinsulfon- oder Sulfanilsäure oder anderen sauren organischen Verbindungen wie Ascorbinsäure.

Die neuen Verbindungen der vorliegenden Erfindung können in Form von Gemischen von Isomeren, wie Stellungsisomeren oder deren Racematen, oder von reinen Racematen oder optisch aktiven Antipoden, vorliegen.

Die Verbindungen der allgemeinen Formel I und ihre Säureadditionssalze besitzen wertvolle pharmakologische, insbesondere antidepressive Eigenschaften. Wie aus den Resultaten der isotopischen Bestimmung der Enzymaktivität hervorgeht, hemmen sie nach oraler Verabreichung an Ratten in Dosen ab 0,3 mg/kg die Monoaminoxidase, insbesondere selektiv und ohne Kumulierung reversibel deren A-Form. Zugleich antagonisieren die Verbindungen der allgemeinen Formel I und ihre pharmazeutisch annehmbaren Säureadditionssalze bei intraperitonealer Verabreichung an der Maus die durch Reserpin bewirkte Hypothermie in Dosen ab 0,3 mg/kg, und die durch Reserpin bewirkte Ptosis, sowie die durch Tetrabenazin bewirkte Katalepsie in Dosen ab je ca. 1 mg/kg. Zusammen mit einem günstigen therapeutischen Index charakterisieren die obgenannten pharmakologischen Eigenschaften die Verbindungen der allgemeinen Formel I und

ihre pharmazeutisch annehmbaren, einsäurigen Säureadditionssalze als Antidepressiva, die, z.B. oral oder parenteral, zur Behandlung von Gemütsdepressionen verabreicht werden können.

Die Erfindung betrifft insbesondere Verbindungen der Formel I, worin $R_1$ Wasserstoff, Niederalkyl oder Niederalkenyl mit bis zu 7 Kohlenstoffatomen, 2-Propinyl, Cycloalkylniederalkyl mit 4-10 Kohlenstoffatomen, Hydroxyniederalkyl mit 2 - 7 Kohlenstoffatomen, oder gegebenenfalls durch Niederalkyl oder Niederalkoxy mit bis zu 7 Kohlenstoffatomen oder durch Halogen substituiertes Benzyl, $R_2$ Wasserstoff oder Niederalkyl mit bis zu 7 Kohlenstoffatomen, $R_3$ Wasserstoff oder Niederalkyl mit bis zu 7 Kohlenstoffatomen bedeutet, $R_4$ und $R_5$ Niederalkyl mit bis zu 7 Kohlenstoffatomen, und $R_6$ und $R_7$ Wasserstoff, Niederalkyl oder Niederalkoxy mit bis zu 7 Kohlenstoffatomen oder Halogen darstellen, und Salze, insbesondere Säureadditionssalze, in erster Linie pharmazeutisch verwendbare, nicht-toxische Säureadditionssalze davon.

Die Erfindung betrifft vor allem Verbindungen der Formel I, worin $R_1$ Wasserstoff, Niederalkyl oder Niederalkenyl mit bis 4 Kohlenstoffatomen, z.B. Methyl oder Allyl, 2-Propinyl, Cycloalkylniederalkyl mit 4-8 Kohlenstoffatomen, z.B. Cyclopropylmethyl, Cyclopentylmethyl, Cyclohexylmethyl, Cyclohexyläthyl oder Cycloheptylmethyl, Hydroxyniederalkyl mit 2-4 Kohlenstoffatomen, z.B. 2-Hydroxyäthyl, 2- oder 3-Hydroxypropyl, oder gegebenenfalls durch Niederalkyl oder Niederalkoxy mit bis zu 4 Kohlenstoffatomen oder durch Halogen substituiertes Benzyl, $R_2$ Wasserstoff oder Niederalkyl mit bis zu 4 Kohlenstoffatomen, $R_3$ Wasserstoff oder Niederalkyl mit bis zu 4 Kohlenstoffatomen bedeutet, $R_4$

und $R_5$ Niederalkyl mit bis zu 4 Kohlenstoffatomen und $R_6$ und $R_7$ Wasserstoff, Niederalkyl oder Niederalkoxy mit bis zu 4 Kohlenstoffatomen oder Halogen darstellen, und Salze, insbesondere Säureadditionssalze, in erster Linie pharmazeutisch verwendbare, nicht-toxische Säureadditionssalze davon.

Die Erfindung betrifft in erster Linie Verbindungen der Formel I, worin $R_1$ insbesondere Wasserstoff, aber auch Methyl, 2-Propinyl, 2-Hydroxyäthyl, Benzyl oder p-Fluorbenzyl, $R_2$ insbesondere Wasserstoff und Methyl, $R_3$ Wasserstoff oder insbesondere Methyl, $R_4$ und $R_5$ Methyl, $R_6$ Methyl oder Wasserstoff und $R_7$ Wasserstoff darstellen, wobei die Gruppen $R_4$ und $R_5$ vorzugsweise die 5- und 7-Stellungen einnehmen, und Salze, insbesondere Säureadditionssalze, in erster Linie pharmazeutisch verwendbare, nicht-toxische Säureadditionssalze davon.

Insbesondere betrifft die Erfindung die neuen, in den Beispielen beschriebenen Verbindungen, und Salze, insbesondere Säureadditionssalze, in erster Linie pharmazeutisch verwendbare, nicht-toxische Säureadditionssalze davon.

Die neuen Verbindungen der Formel I können nach an sich bekannten Methoden hergestellt werden.

So kann man sie erhalten, wenn man eine Verbindung der Formel

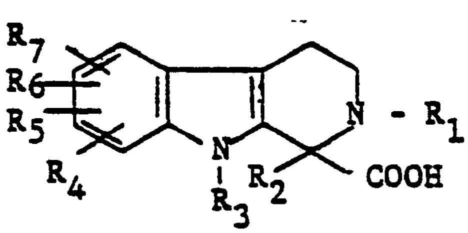

(II)

decarboxyliert, und, wenn erwünscht, eine verfahrensgemäss erhaltene Verbindung der Formel I in eine andere Verbindung der Formel I umwandelt, und/oder, wenn erwünscht, ein verfahrensgemäss erhaltenes Salz in die freie Verbindung oder in ein anderes Salz oder eine verfahrensgemäss erhaltene freie Verbindung in ein Salz umwandelt und/oder wenn erwünscht, ein verfahrensgemäss erhaltenes Isomerengemisch in die einzelnen Isomeren trennt.

Die Decarboxylierung einer Säure-Verbindung der Formel II erfolgt vorteilhafterweise durch Erwärmen, vorzugsweise in einem Bereich von 40 - 100°C, und/oder Behandlung mit einer wässerigen Säure, wie mit einer Mineralsäure, z.B. Salzsäure, oder einer starken organischen Säure, wie Methansulfonsäure.

Die Ausgangsstoffe der Formel II sind neu. Sie lassen sich jedoch in an sich bekannter Weise herstellen, z.B. indem man zunächst in einer Verbindung der Formel

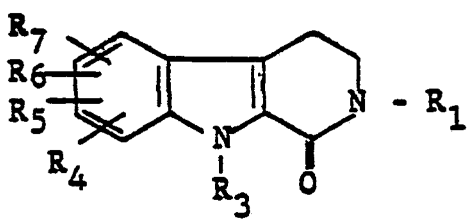

(III)

eine Spaltung der Amidbindung vornimmt. Diese erfolgt vorzugsweise mit einer anorganischen Base, z.B. Kalilauge in einem organischen Lösungsmittel, z.B. Aethylalkohol. Die erhaltene Aminocarbonsäure führt nach Decarboxylierung mit einer wässerigen Säure, wie z.B. Salzsäure oder Methansulfonsäure zu einer Verbindung der Formel

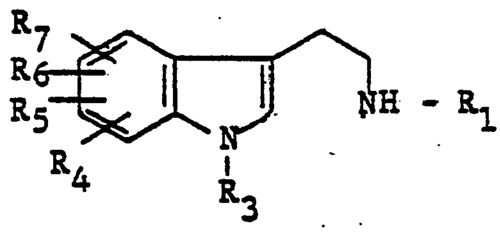

(IV)

Die Verbindung IV wird dann mit einer Verbindung der Formel $R_2$ - CO - COOH (V) unter Säureeinfluss in der Kälte zu der entsprechenden Verbindung der Formel II umgesetzt.

Die Verbindungen der Formel III sind ebenfalls neu. Sie lassen sich jedoch auch in an sich bekannter Weise herstellen.

Eine Möglichkeit zur Herstellung von Verbindungen der Formel III, in der $R_3$ Wasserstoff bedeutet, besteht darin, dass ein definitionsgemäss ring-substituiertes Anilin zuerst diazotiert und dann mit einem gegebenenfalls entsprechend der Definition von $R_1$ N-substituierten 2-Oxo-3-piperidincarbonsäureester zum entsprechenden 2,3-Piperidindion-3-hydrazon gekuppelt wird, das dann durch Erhitzen in Ameisensäure in die entsprechenden Verbindungen der Formel III übergeführt wird.

Eine weitere Möglichkeit zur Herstellung solcher Verbindungen besteht darin, dass ein definitionsgemäss ringsubstituiertes diazotiertes Anilin mit Monoäthyl-2-(3-chlorpropyl)-malonat gekuppelt und das entstehende Hydrazon durch Kochen in einem Alkohol, vorzugsweise n-Butanol, in salzsaurem Milieu in das entsprechende, im aromatischen Ring substituierte 2-Aethoxycarbonyltryptaminhydrochlorid überführt. Durch Freisetzen der Base und Erhitzen bildet sich dann die entsprechende Verbindung

der Formel III.

Verbindungen der Formel III, in welchen $R_3$ Niederalkyl darstellt, können aus den entsprechenden N-unsubstituierten Verbindungen z.B. durch Behandeln mit Diniederalkylsulfat in alkalischer wässeriger Lösung hergestellt werden.

Verbindungen der Formel III, in welchen $R_1$ und $R_3$ Niederalkyl darstellen, lassen sich durch gleichzeitige Alkylierung an den beiden Stickstoffatomen aus entsprechendem N-unsubstituierten Verbindungen herstellen, z.B. durch Behandeln mit Natriumhydrid und Niederalkylbromid in Toluol oder durch Behandeln mit Diniederalkylsulfat in Methylenchlorid, Natronlauge und Tetrabutylammoniumbromid.

Die oben erwähnte erfindungsgemässe Umsetzung kann auch so durchgeführt werden, dass auf eine Erfassung der Verbindung der Formel II verzichtet wird, d.h. eine Verbindung der Formel IV wird ebenfalls unter Säureeinfluss mit einer Verbindung der Formel V, aber unter energischen Bedingungen, also z.B. unter Erhitzen umgesetzt, wobei die Umsetzung und die Decarboxylierung in einem Arbeitsgang erfolgen.

Die neuen Verbindungen können ebenfalls erhalten werden, indem man eine Verbindung der Formel

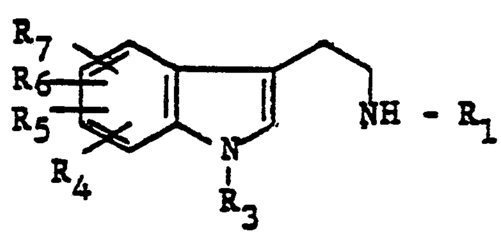

(IV)

mit einer Verbindung der Formel

$R_2$ - CHO (VI)

umsetzt, und, wenn erwünscht, die zusätzlichen Massnahmen durchführt.

Die Umsetzung einer Verbindung der Formel IV mit einer Verbindung VI erfolgt vorteilhafterweise unter Einfluss einer Säure, wie einer Mineralsäure, z.B. Salzsäure oder einer organischen Säure, z.B. p-Toluolsulfonsäure, in der Kälte in einem protischen Lösungsmittel, wie z.B. Wasser oder Aethylalkohol.

Die Ausgangsstoffe der Formel IV lassen sich in an sich bekannte Weise, z.B. wie oben beschrieben, herstellen.

Die neuen Verbindungen können ebenfalls erhalten werden, indem man in einer Verbindung der Formel

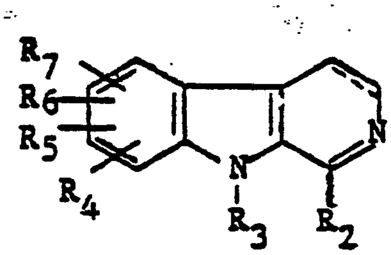

(VII)

oder

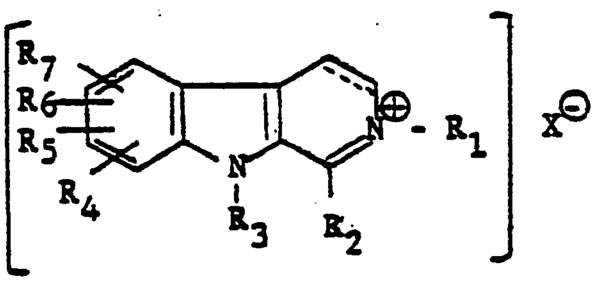

(VIII)

die zusätzlich eine Doppelbindung in 3,4-Stellung aufweisen kann, worin $X^{\ominus}$ ein Anion bedeutet, die C=N-Doppelbindung und eine gegebenenfalls vorhandene 3,4-Doppelbindung reduziert, und, wenn erwünscht, die zusätzlichen Massnahmen durchführt.

Das Anion ist vorzugsweise dasjenige einer Halogenwasserstoffsäure.

Die Reduktion der Verbindungen der Formeln VII und VIII kann nach an sich bekannten Methoden erfolgen, und zwar entweder mit katalytisch aktiviertem Wasserstoff oder mit nascierendem Wasserstoff. Als Katalysatoren eignen sich z.B. Edelmetallkatalysatoren, wie Palladium auf Kohle. Für die Reduktion mit nascierendem Wasserstoff eignen sich z.B. Natrium und ein niederer Alkohol, wie Aethanol, Butanol oder Amylalkohol. Ferner eignen sich unedle Metalle, wie z.B. Zink und Säuren, wie Salzsäure oder Essigsäure. Weiter können Natriumamalgam und eine Mineralsäure verwendet werden. Ein weiteres Reduktionsmittel ist ein Hydridreduktionsmittel, wie z.B. Natriumborcyanhydrid in Acetonitril. Besonders eignet sich Natriumborhydrid in einem gegebenenfalls mit Wasser gemischten niederen Alkanol, wie Methanol oder Aethanol.

Die Ausgangsstoffe der Formeln VII und VIII sind neu. Sie lassen sich in an sich bekannter Weise herstellen, z.B. indem man eine Verbindung der Formel

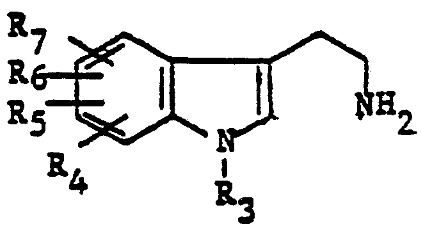

(IX)

mit einer Verbindung der Formel $R_2COHal$ (Xa) oder der Formel $(R_2CO)_2O$ (Xb) umsetzt, worin Hal ein Halogenatom, vorzugsweise Chlor, bedeutet. Das entstandene Säureamid wird durch Umsetzen z.B. mit Phosphortrichlorid in Toluol oder mit Polyphosphorsäureester in Chloroform oder mit Polyphosphorsäure zur Verbindung der Formel

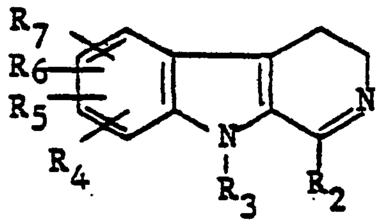

(VIIa)

cyclisiert, die direkt oder nach Quaternisierung mit einer Verbindung der Formel $R_1$ - X (XI) im erfindungsgemässen Verfahren verwendet wird.

Die neuen Verbindungen können ebenfalls erhalten werden, indem man eine Verbindung der Formel

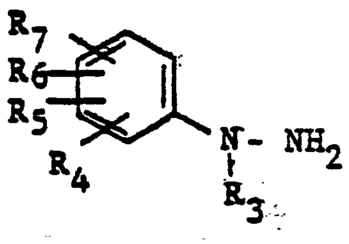

(XII)

mit einer Verbindung der Formel

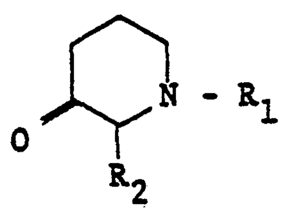

(XIII)

oder mit dem Hydrat oder einem Ketal derselben umsetzt, und, wenn erwünscht, die zusätzlichen Massnahmen durchführt.

Als Ketale kommen beispielsweise die Dimethyl- oder Diäthylketale und vor allem die Aethylenketale in Betracht.

Die Umsetzung einer Verbindung der Formel XII mit einer Verbindung der Formel XIII erfolgt vorteilhafterweise in saurem Medium, wie in Gegenwart von Polyphosphorsäure, oder Lewis-Säuren, insbesondere Bortrifluorid-ätherat in Eisessig oder äthanolischer Salzsäure oder verdünnter wässeriger Schwefelsäure in der Wärme vorzugsweise bei einer Temperatur von 40 - 150°C. Unter den gleichen Reaktionsbedingungen können auch Ketale umgesetzt werden.

Die Ausgangsstoffe sind bekannt oder lassen sich in an sich bekannter Weise herstellen.

Die neuen Verbindungen können ebenfalls erhalten werden, indem man in einer Verbindung der Formel

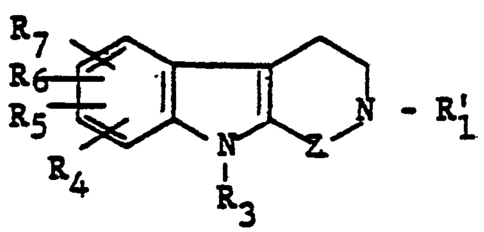

(XIV)

worin

Z die -CO- oder die $>CH-R_2$-Gruppe bedeutet und

$R_1'$ entweder $R_1$ oder die $-CO-R_1^a$-Gruppe darstellt, wobei

$R_1^a$ einen um eine Methylengruppe verminderten, sonst aber der oben angegebenen Definition für $R_1$ entsprechenden Rest bedeutet,

wobei mindestens eines der Symbole Z und $R_1'$ eine Carbonylgruppe darstellt bzw. enthält,

vorhandene Carbonylgruppen zu Methylengruppen reduziert, und, wenn erwünscht, die zusätzlichen Massnahmen durchführt.

Erfindungsgemäss werden insbesondere Verbindungen der Formel XIV reduziert, in welcher Z die -CO-Gruppe darstellt und $R_1'$ die Bedeutung von $R_1$ hat.

Als Reduktionsmittel eignen sich besonders komplexe Metallhydride, wie Lithiumaluminiumhydrid und Natriumaluminiumborhydrid in Gegenwart eines Lösungsmittels, wie z.B. Tetrahydrofuran, Dimethoxyäthan, Diäthyläther, Toluol, Benzol und insbesondere Dioxan. Die Reduktion wird vorzugsweise unter leichtem Erwärmen durchgeführt. Ein weiteres geeignetes Reduktionsmittel ist Natrium in Butanol.

Die Ausgangsstoffe der Formel XIV sind neu. Sie lassen sich jedoch in an sich bekannter Weise herstellen.

Eine Möglichkeit zur Herstellung von Ausgangsstoffen der Formel XIV, insbesondere solchen der von dieser umfassten Formel III, in der $R_3$ Wasserstoff bedeutet, besteht darin, dass ein definitionsgemäss ringsubstituiertes Anilin zuerst diazotiert und dann mit einem gegebenenfalls N-substituierten 2-Oxo-3-piperidincarbonsäureester zum entsprechenden 2,3-Piperidindion-3-hydrazon gekuppelt wird, das dann durch Erhitzen in Ameisensäure in den entsprechenden Ausgangsstoff der Formel III übergeführt wird.

Eine weitere Möglichkeit zur Herstellung solcher Ausgangsstoffe besteht darin, dass ein definitionsgemäss ringsubstituiertes diazotiertes Anilin mit Monoäthyl-2-(3-chlorpropyl-malonat gekuppelt und das entstehende Hydrazon durch Kochen in einem Alkohol, vorzugsweise n-Butanol, in salzsaurem Milieu in das entsprechende, definitionsgemäss im aromatischen Ring substituierte 2-Aethoxycarbonyl-tryptaminhydrochlorid überführt. Durch Freisetzen der Base und Erhitzen bildet sich dann der entsprechende Ausgangsstoff der Formel III.

Ausgangsstoffe der Formel III, in welcher $R_3$

Niederalkyl darstellt, können aus den entsprechenden N-unsubstituierten Ausgangsstoffen, z.B. durch Behandeln mit Diniederalkylsulfat in alkalischer wässeriger Lösung, hergestellt werden.

Ausgangsstoffe der Formel III, in welcher $R_1$ und $R_3$ Niederalkyl darstellen, lassen sich durch 2-fache Alkylierung aus entsprechenden N-unsubstituierten Ausgangsstoffen herstellen, z.B. entweder durch Behandeln mit Natriumhydrid und Niederalkylbromid in Toluol oder durch Behandeln mit Diniederalkylsulfat in Methylenchlorid, Natronlauge und Tetrabutylammoniumbromid.

Weiter können erfindungsgemäss Ausgangsstoffe der Formel XIV, in welchen Z die -CO-Gruppe darstellt und $R_1'$ die CO-$R_1$-Gruppe bedeutet, reduziert werden. Als Reduktionsmittel eignen sich besonders komplexe Metallhydride, vorzugsweise Lithiumaluminiumhydrid in Gegenwart von Tetrahydrofuran als Lösungsmittel.

Ausgangsstoffe der Formel XIV, in welcher Z die -CO-Gruppe und $R_1'$ die -CO-$R_1^a$-Gruppe darstellen, können z.B. durch Acylieren von entsprechenden Ausgangsstoffen gewonnen werden, in denen $R_1'$ Wasserstoff bedeutet.

Die neuen Verbindungen der Formel I, in denen $R_1$ und/oder $R_3$ Wasserstoff bedeutet, können ebenfalls erhalten werden, indem man eine entsprechende Verbindung, in der an Stelle von $R_1$ und/oder $R_3$ eine durch Solvolyse oder durch Reduktion abspaltbare Schutzgruppe vorliegt, diese durch Solvolyse bzw. Reduktion entfernt.

Beispielsweise werden Acylgruppen, wie die Acetylgruppe oder Niederalkoxycarbonylgruppen durch Hydrolyse in alkalischen oder saurem Medium, oder die

Benzyl- oder Benzyloxycarbonylgruppen durch Reduktion, insbesondere katalytische Hydrierung, abgespalten.

Die Ausgangsstoffe, in denen an Stelle von $R_1$ eine Acylgruppe vorliegt und $R_3$ Niederalkyl bedeutet, lassen sich beispielsweise aus Verbindungen der Formel I, in denen $R_1$ und $R_3$ Wasserstoff bedeuten, durch Acylierung am basischen Stickstoffatom und anschliessende Einführung eines Niederalkylrestes $R_3$ auf die oben angegebene Weise herstellen, z.B. entweder durch Behandeln mit Natriumhydrid in Toluol oder durch Behandeln mit Diniederalkylsulfat in Methylenchlorid.

Ausgangsstoffe die als Schutzgruppe in 2-Stellung eine gegebenenfalls substituierte Benzylgruppe und als $R_3$ Niederalkyl enthalten, fallen unter die Formel I und können nach einem der vor- oder nachstehend beschriebenen Verfahren hergestellt werden.

Ausgangsstoffe, die in 9-Stellung an Stelle von $R_3$ einen gegebenenfalls substituierten Benzylrest als hydrogenolytisch abspaltbare Schutzgruppe enthalten, kann man herstellen, indem man zur Umsetzung mit einer Verbindung der weiter oben angegebenen Formel XIII anstelle eines Phenylhydrazins der Formel XII eine analoge Verbindung verwendet, die an Stelle von $R_3$ einen gegebenenfalls substituierten Benzylrest enthält und im Reaktionsprodukt gegebenenfalls ein Wasserstoffatom $R_1$ auf die weiter unten angegebene Weise durch einen anderen Rest $R_1$ ersetzt.

Verfahrensgemäss erhältliche Verbindungen der Formel I können in andere Verbindungen der Formel I übergeführt werden.

So kann man in Verbindungen der Formel I, in

denen $R_1$ Wasserstoff bedeutet, diesen Wasserstoff durch einen Substituenten ersetzen. Dieser Ersatz kann dadurch erfolgen, dass man eine solche Verbindung der Formel I mit einer Verbindung der Formel $R_1$-Y (XV), worin Y reaktionsfähiges verestertes Hydroxy bedeutet, umsetzt. Als Säurerest kommt insbesondere Halogen in Frage. Dieser Ersatz kann weiter auch dadurch erfolgen, dass man eine solche Verbindung der Formel I mit einer Oxoverbindung der Formel $R_1^b$=O, (XVI) in welcher $R_1^b$ den einem Rest $R_1$ entsprechenden geminal zweiwertigen Rest bedeutet, unter reduzierenden Bedingungen umsetzt. Als Reduktionsmittel eignen sich Natriumborcyanhydrid in Acetonitril, ferner Natriumborhydrid in einem gegebenenfalls mit Wasser gemischten niederen Alkohol, wie Methanol oder katalytisch aktiviertem Wasserstoff.

Ferner kann man Verbindungen der Formel I, in denen $R_1$ die Benzylgruppe bedeutet, durch Reduktion in Verbindungen der Formel I überführen, in denen $R_1$ Wasserstoff bedeutet. Diese Reduktion wird vorzugsweise katalytisch vorgenommen.

Ferner kann man Verbindungen der Formel I, in denen $R_3$ Wasserstoff bedeutet, durch Alkylierung in Verbindungen der Formel I überführen, in denen $R_3$ Niederalkyl bedeutet. Diese Ueberführung kann dadurch erfolgen, dass man eine Verbindung der Formel I, in der $R_3$ Wasserstoff bedeutet mit einer Verbindung der Formel $R_3$-Y (XVII), worin Y reaktionsfähig verestertes Hydroxy bedeutet, umsetzt. Als Säurereste kommen besonders diejenigen einer Halogenwasserstoffsäure oder der Schwefelsäure in Frage. Diese Umsetzung kann z.B. in Gegenwart von Natriumamid und flüssigem Ammoniak, n-Butyl-lithium in Tetrahydrofuran oder in Gegenwart von Natriumhydrid und Toluol oder in Gegenwart von Methylenchlorid und 50%-iger Natronlauge und Tetrabutylammoniumbromid, oder Tetrabutylammoniumhydrogensulfat durchgeführt werden. Falls in der erhaltenen Verbindung der Formel I $R_1$ Wasserstoff bedeuten soll, so ist es zweckmässig das entsprechende Stickstoffatom während der Alkylierung durch eine Acetylgruppe zu schützen.

Die obigen Reaktionen werden in üblicher Weise in An- oder Abwesenheit von Verdünnungs-, Kondensations- und/oder katalytischen Mitteln, falls notwendig, bei erniedrigter oder erhöhter Temperatur, im geschlossenen Gefäss und/oder einer Inertgasatmosphäre durchgeführt.

Je nach Verfahrensbedingungen und Ausgangsstoffen erhält man gegebenenfalls salzbildende Endstoffe in freier Form oder in Form ihrer Salze, die sich in üblicher Weise ineinander oder in andere Salze umwandeln lassen. Verbindungen der Formel I mit basischem Charakter kann man gegebenenfalls in Form ihrer Säureadditionssalze erhalten. Letztere lassen sich durch Umsetzen einer freien basischen Verbindung der Formel I mit einer organischen oder anorganischen Säure, insbesondere solchen, die zur Bildung von pharmazeutisch verwendbaren Salze geeignet sind, in Salze umwandeln. Erhaltene Säureadditionssalze von basischen Verbindungen der Formel I können in an sich bekannter Weise, z.B. durch Behandeln mit alkalischen Mitteln, z.B. Alkalimetallhydroxiden oder basischen Ionenaustauschern, in die freien Basen überführt werden.

Diese und andere Salze können zur Reinigung der neuen Verbindungen verwendet werden, z.B. indem man die freien Verbindungen in ihre Salze überführt, diese isoliert und wieder in die freien Verbindungen überführt.

Infolge der engen Beziehung zwischen den neuen Verbindungen in freier Form und in Form ihrer Salze sind im vorausgegangenen und nachfolgend unter den freien Verbindungen sinn- und zweckmässig gegebenenfalls auch die entsprechenden Salze zu verstehen.

Die neuen Verbindungen können je nach Wahl der Ausgangsstoffe und Arbeitsweisen, als optische Antipoden oder Racemate, oder sofern sie mindestens zwei asymmetrische Kohlenstoffatome enthalten, auch als Racematgemische oder gegebenenfalls auch als Gemische von Stellungsisomeren vorliegen.

Erhaltene Racematgemische können auf Grund der physikalisch-chemischen Unterschiede der Diastereoisomeren in bekannter Weise, z.B. durch Chromatographie und/oder fraktionierte Kristallisation, in die beiden stereoisomeren (diastereomeren) Racemate aufgetrennt werden. Ebenfalls vorzugsweise durch fraktionierte Kristallisation können Gemische von Stellungsisomeren getrennt werden.

Erhaltene Racemate lassen sich nach an sich bekannten Methoden in die Antipoden zerlegen, z.B. durch Umkristallisieren aus einem optisch aktiven Lösungsmittel, durch Behandeln mit geeigneten Mikroorganismen oder durch Umsetzen mit einer, mit der racemischen Verbindung Salze bildenden optisch aktiven Substanz, insbesondere Säure, und Trennen des auf diese Weise erhaltenen Salzgemisches, z.B. auf Grund von verschiedenen Löslichkeiten, in die diastereomeren Salze, aus denen die freien Antipoden durch Einwirkung geeigneter Mittel freigesetzt werden können. Besonders gebräuchliche, optisch aktive Säuren sind z.B. die D- und L-Formen von Weinsäure, Di-o-Toluylweinsäure, Apfelsäure, Mandelsäure, Camphersulfonsäure, Glutaminsäure, Asparaginsäure oder Chinasäure. Vorteilhaft isoliert man den wirksameren der beiden Antipoden.

Die Erfindung betrifft auch diejenigen Ausführungsformen des Verfahrens, nach denen man von einer, auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht, oder bei denen man einen Ausgangsstoff unter den Reaktionsbedingungen bildet, oder bei denen eine Reaktionskomponente gegebenenfalls in Form eines Derivates, z.B. eines Salzes vorliegt.

Zweckmässig verwendet man für die Durchführung der erfindungsgemässen Reaktionen solche Ausgangsstoffe, die zu den eingangs besonders erwähnten Gruppen von Endstoffen und besonders zu den speziell beschriebenen oder hervorgehobenen Endstoffen führen.

Die Verbindungen der allgemeinen Formel I sowie ihre pharmazeutisch annehmbaren Säureadditionssalze werden vorzugsweise peroral oder rektal verabreicht, können jedoch in Form wässriger Lösungen ihrer Säureadditionssalze auch parenteral verabreicht werden.

Die täglichen Dosen für Warmblüter bewegen sich zwischen 0,03 und 3 mg/kg und liegen für Warmblüter von ca. 70 kg Körpergewicht vorzugsweise zwischen 3 und 30 mg. Geeignete Doseneinheitsformen, wie Dragées, Tabletten oder Suppositorien, enthalten vorzugsweise 1 bis 30 mg eines erfindungsgemässen Wirkstoffes, d.h. einer Verbindung der allgemeinen Formel I oder eines pharmazeutisch annehmbaren Säureadditionssalzes einer solchen. Zu ihrer Herstellung kombiniert man den Wirkstoff mit festen pulverförmigen Trägerstoffen, wie Lactose, Saccharose, Sorbit, Mannit, Stärken, wie Kartoffelstärke, Maisstärke oder Amylopektin, ferner Laminariapulver oder Citruspulpenpulver, Cellulosederivaten oder Gelatine, gegebenenfalls unter Zusatz von Gleitmitteln, wie Magnesium- oder Calciumstearat oder Polyäthylenglkolen, zu Tabletten oder zu Dragées-Kernen. Letztere überzieht man beispielsweise mit konzentrierten Zuckerlösungen, welche z.B. noch arabischen Gummi, Talk und/oder Titandioxid enthalten können, oder mit einem in leichtflüchtigen organischen Lösungsmitteln oder Lösungsmittelgemischen gelösten Lack. Diesen Ueberzügen können Farbstoffe zugefügt werden, z.B. zur Kennzeichnung verschiedener Wirkstoffdosen. Als weitere orale Doseneinheitsformen eignen sich Steckkapseln aus Gelatine sowie weiche, geschlossene Kapseln aus Gelatine und einem Weichmacher, wie Glycerin. Die ersten enthalten den Wirkstoff vorzugsweise als Granulat in Mischung mit Gleitmitteln, wie Talk oder Magnesiumstearat, und gegebenenfalls Stabilisatoren, wie Natriummetabisulfit oder Ascorbinsäure.

Die folgenden Vorschriften sollen die Herstellung von Tabletten, Dragées, Suppositorien und Ampullen näher erläutern:

a) 100,0 g 1,5,7,9-Tetramethyl-2,3,4,9-tetrahydro-1H-pyrido[3,4-b]indol-maleat werden mit 450 g Lactose und 292 g Kartoffelstärke vermischt, die Mischung mit einer alkoholischen Lösung von 8 g Gelatine befeuchtet und durch ein Sieb granuliert. Nach dem Trocknen mischt man 60 g Kartoffelstärke, 60 g Talk, 10 g Magnesiumstearat und 20 g hochdisperses Siliciumdioxid zu und presst die Mischung zu 10'000 Tabletten von je 100 mg Gewicht und 10 mg Wirkstoffgehalt, die gewünschtenfalls mit Teilkerben zur feineren Anpassung der Dosierung versehen sein können.

b) 12,5 g 5,7,9-Trimethyl-2,3,4,9-tetrahydro-1H-pyrido[3,4-b]indol-methansulfonat werden mit 16 g Maisstärke und 6 g hochdispersem Siliciumdioxid gut vermischt. Die Mischung wird mit einer Lösung in ca. 70 ml Isopropylalkohol befeuchtet und durch ein Sieb mit 1,2 mm Maschenweite granuliert. Das Granulat wird ca. 14 Stunden getrocknet und dann durch ein Sieb mit 1,2 bis 1,5 mm Maschenweite geschlagen. Hierauf wird es mit 16 g Maisstärke, 16 g Talk und 2 g Magnesiumstearat vermischt und zu 1000 Dragée-Kernen gepresst. Diese werden mit einem konzentrierten Sirup von 2 g Lacca, 7,5 g arabischen Gummi, 0,15 g Farbstoff, 2 g hochdispersem Siliciumdioxid, 25 g Talk und 53,35 g Zucker überzogen und getrocknet. Die erhaltenen Dragées wiegen je 172,5 mg und enthalten je 12,5 mg Wirkstoff.

c) 25,0 g 1,5,7,9-Tetramethyl-2,3,4,9-tetrahydro-9H-pyrido[3,4-b]indol-maleat und 1975 g fein geriebene Suppositoriengrundmasse (z.B. Kakaobutter) werden gründlich gemischt und dann geschmolzen. Aus der durch Rühren homogen gehaltenen Schmelze werden 1000 Suppositorien von 2 g gegossen. Sie enthalten je 25 mg Wirkstoff.

d) 12,5 g 5,7,9-Trimethyl-2,3,4,9-tetrahydro-9H-pyrido[3,4-b]indol-methansulfonat werden in 1 Liter bidestilliertem pyrogenfreiem Wasser gelöst und die Lösung in 1000 Ampullen abgefüllt und sterilisiert. Eine Ampulle enthält eine 2,5%-ige Lösung von 12,5 mg Wirkstoff.

Die nachfolgenden Beispiele erläutern die Herstellung der neuen Verbindungen der allgemeinen Formel I und von bisher nicht bekannten Ausgangsstoffen näher,

sollen jedoch den Umfang der Erfindung in keiner Weise beschränken. Die Temperaturen sind in Celsiusgraden angegeben.

Beispiel 1

14,6 g 6,7-Dimethyl-2,3,4,9-tetrahydro-1H-pyrido[3,4-b]indol-1-carbonsäure werden bei Rückflusstemperatur portionenweise in 350 ml Wasser und 50 ml konz. Salzsäure eingetragen. Das Reaktionsgemisch wird 2 Stunden bei Rückflusstemperatur gehalten und nach erfolgter Abkühlung die klare Lösung mit ca. 70 ml 2-n. Natronlauge alkalisch gestellt. Das ausgefallene Produkt wird mit Chloroform ausgeschüttelt, mit Wasser gewaschen und die organische Phase über Magnesiumsulfat getrocknet. Nach Entfernen des Lösungsmittel am Vakuum verbleibt ein kristalliner Rückstand, welcher nach Umkristallisation aus 90%-igem Alkohol reines 6,7-Dimethyl-2,3,4,9-tetrahydro-1H-pyrido[3,4-b]indol vom Smp. 252 - 256° liefert.

20,0 g 6,7-Dimethyl-2,3,4,9-tetrahydro-1H-pyrido[3,4-b]indol werden in 200 ml abs. Alkohol gelöst und mit 9,6 g Methansulfosäure tropfenweise versetzt. Das ausgefallene Produkt wird abfiltriert und aus 96%-igem Alkohol umkristallisiert. Das reine 6,7-Dimethyl-2,3,4,5-tetrahydro-1H-pyrido[3,4-b]indol-methansulfonat schmilzt bei 304 - 306°.

Das Ausgangsmaterial kann auf folgende Weise hergestellt werden:

a) 25,5 g 2-Oxo-3-piperidincarbonsäure-äthylester werden mit einer Lösung von 9,0 g Kaliumhydroxyd in 300 ml Wasser 12 Stunden bei 30° gerührt. Die erhaltene Lösung wird mit Eis-Natriumchlorid-Mischung auf 0° gekühlt und mit 1,0 n Salzsäure auf pH = 4,5 gestellt. Anschliessend wird eine frisch bereitete Diazolösung, hergestellt aus 18,8 g 3,4-Dimethylanilin, 11,25 g Natriumnitrit und 350 ml 1-n. Salzsäure, welche mit 10%-iger Natriumcarbonatlösung auf pH = 6,0 gestellt wurde, im Verlaufe von ca. 10 Minuten bei 0° eingetragen. Das Reaktionsgemisch wird 1 Stunde bei 5-10° gerührt und mit 45%-iger Natriumacetatlösung auf pH = 5,5 gepuffert. Das Gemisch wird 12 Stunden bei Raumtemperatur gerührt, erneut auf 0° gekühlt und das ausgefallene Produkt abfiltriert. Nach Waschen mit Eiswasser wird das Rohprodukt im Vakuum bei 60° getrocknet. Es schmilzt bei 214 - 219°. Eine Probe wird aus Alkohol umkristallisiert und ergibt das reine 2,3-Piperidindion-3-[(3,4-dimethylphenyl)-hydrazon] vom Smp. 237-239°.

b) 29,2 g 2,3-Piperidindion-3-[(3,4-dimethylphenyl)]-hydrazon werden in 200 ml 70%-iger Ameisensäure 1 Stunde am Rückfluss gekocht. Das Reaktionsgemisch wird mit 500 ml Wasser verdünnt und mit Eis auf +5° gekühlt. Das ausgefallene Produkt wird abfiltriert. Nach Waschen mit Eiswasser und Trocknen im Vakuum bei 50° wird das Rohprodukt aus absolutem Aethanol umkristallisiert und ergibt ein Gemisch von 5,6- und 6,7-Dimethyl-2,3,4,9-tetrahydro-1H-pyrido[3,4-b]indol-1-on vom Smp. 220 - 250°.

c) 21,4 g des gemäss b) erhaltenen Gemisches werden mit 46 g Kaliumhydroxyd in 190 ml Wasser und 190 ml Aethanol 12 Stunden unter Rückfluss gekocht. Anschliessend werden 200 ml Wasser beigefügt und der Alkohol weitgehend abdestilliert. In das heisse klare

Reaktionsgemisch werden 100 ml konz. Salzsäure im Verlaufe von 40 Minuten langsam eingetropft. Die klare gelbe Lösung wird langsam bräunlich und schäumt stark. Nach beendigter Säurezugabe wird das Gemisch 2 Stunden unter Rückfluss gehalten, abgekühlt und mit konz. Natronlauge alkalisch gestellt und die erhaltene milchige Suspension mit Chloroform ausgeschüttelt. Die organische Phase wird mit Wasser gewaschen, über Magnesiumsulfat getrocknet und am Vakuum zur Trockne eingedampft. Den erhaltenen rohen Rückstand vom Smp. 75 - 85° besteht aus einem Gemisch von 4,5-Dimethyl- und 5,6-Dimethyl-tryptamin.

d) 188 g des gemäss c) erhaltenen rohen Gemisches werden im Kugelrohr unter Hochvakuum destilliert. (Kp 0,01 mm Hg = 145-155°). Das erhaltene Destillat wird zur weiteren Reinigung aus Benzol umkristallisiert. Dabei wird gereinigtes Gemisch von 4,5-Dimethyl- und 5,6-Dimethyltryptamin erhalten; Smp. 92 - 106°. 150 g des gereinigten Gemisches werden in 750 ml absolutem Aethanol gelöst und eine Lösung von 200 g (+)-Campher-10-sulfon-säure-monohydrat in 750 ml absolutem Aethanol beigefügt. Nach erfolgter Abkühlung werden 1000 ml Aethyläther beigefügt, um die Kristallisation zu vervollständigen. Das Gemisch wird 12 Stunden im Kühlschrank stehen gelassen, die Kristalle werden abfiltriert und mit wenig Aethylacetat-Aether gewaschen und den Rückstand im Vakuum getrocknet. 310 g der erhaltenen Camphersulfonsäuresalze werden in 4 Liter 90%-igem Aethanol heiss gelöst und bis zur vollständigen Abkühlung auf Raumtemperatur stehengelassen. Das ausgefallene Produkt wird abfiltriert, und zur Gewinnung des 4,5-Dimethyltryptamins aufbewahrt.

Die erhaltene Mutterlauge wird auf 2000 ml eingeengt und im Eisschrank 12 Stunden stehen gelassen. Das erneut ausgefallene Produkt (70 g) wird abfiltriert, zweimal aus 96%-igem Aethanol umkristallisiert und ergibt das reine 5,6-Dimethyl-tryptamin-(+)-campher-10-sulfonat vom Smp. 254-256° in Form von feinen Nädelchen. 58,8 g dieses Salzes werden mit 100 ml 2-n. Natronlauge in 200 ml Methylenchlorid geschüttelt. Die organische Phase wird abgetrennt, mit Wasser gewaschen und über Magnesiumsulfat getrocknet. Nach Entfernen des Lösungsmittels wird der kristalline Rückstand aus Aether-Pentan umkristallisiert und ergibt reines 5,6-Dimethyltryptamin vom Smp. 108 - 109°.

Das bei der 1. Umkristallisation erhaltene kristalline Gemisch der Camphersulfonsäuresalze (250 g) wird erneut in 3 Liter 90%-igem Aethanol heiss gelöst und langsam abgekühlt bis ca. 1/3 der Substanzmenge auskristallisiert hat. Anschliessend wird das Kristallisat sofort abfiltriert und die erhaltene Kristallmasse 3 mal in gleicher Weise umkristallisiert. Es wird reines 4,5-Dimethyl-tryptamin-(+)campher-10-sulfonat vom Smp. 275-277° in der Form von derben Blättchen erhalten. Das Camphersulfonat wird mit 100 ml 2n-Natronlauge in 200 ml Methylenchlorid geschüttelt; die organische Phase wird abgetrennt, mit Wasser gewaschen und über Magnesiumsulfat getrocknet. Nach Entfernen des Lösungsmittels wird der kristalline Rückstand aus Ligroin umkristallisiert und ergibt reines 4,5-Dimethyltryptamin vom Smp. 136-137°.

e) 11,28 g 5,6-Dimethyltryptamin werden in 60 ml 1n-Salzsäure und 250 ml Wasser gelöst und im Verlaufe von 15 Minuten bei Raumtemperatur eine Lösung von 6,07 g Glyoxylsäure-monohydrat in 25 ml Wasser eingetropft. Das

Gemisch wird 30 Minuten bei Raumtemperatur nachgerührt und anschliessend mit 10%-iger Kalilauge auf pH 3,8-4,0 gepuffert. Die erhaltene Suspension wird 30 Minuten bei Raumtemperatur nachgerührt und anschliessend mit Eis auf +5° gekühlt. Das ausgefallene Produkt wird abfiltriert, mit wenig Eiswasser gewaschen und im Vakuum bei 50° getrocknet. Das erhaltene Rohprodukt wird in Aethanol aufgeschlämmt, abfiltriert und erneut getrocknet. Die erhaltene reine 6,7-Dimethyl-2,3,4,9-tetrahydro-1H-pyrido[3,4-b] indol-1-carbonsäure schmilzt bei 243-245°.

Beispiel 2

14,6 g 5,6-Dimethyl-2,3,4,9-tetrahydro-1H-pyrido[3,4-b]indol-1-carbonsäure werden in 350 ml Wasser suspendiert und bei Rückflusstemperatur werden im Verlaufe von ca. 30 Minuten 50 ml konzentrierte Salzsäure eingetropft. Nach beendigter Zugabe wird das Gemisch weitere 3 Stunden unter Rückfluss erhitzt. Nach erfolgter Abkühlung wird das Gemisch mit konzentrierter Natronlauge alkalisch gestellt und mit Chloroform ausgeschüttelt. Die organische Phase wird abgetrennt, mit Wasser gewaschen und über Magnesiumsulfat getrocknet. Nach Entfernung des Lösungsmittels wird der erhaltene kristalline Rückstand aus einem Benzol-Petroläther-Gemisch umkristallisiert und ergibt reines 5,6-Dimethyl-2,3,4,9-tetrahydro-1H-pyrido [3,4-b]indol vom Smp. 203-205°.

Aus der erhaltenen Base wird analog Beispiel 1 mit Methansulfonsäure das Methansulfonat hergestellt. Es wurden 12,5 g des reinen 5,6-Dimethyl-2,3,4,9-tetrahydro-1H-pyrido[3,4-b]indol-methansulfonats vom Smp. 308-310° erhalten.

Das Ausgangsmaterial kann auf folgende Weise hergestellt werden:

a) Analog Beispiel 1e) wird aus 4,5-Dimethyltryptamin und Glyoxylsäuremonohydrat die 5,6-Dimethyl-2,3,4,9-tetrahydro-1H-pyrido[3,4-b]indol-1-carbonsäure vom Smp. 223-224° enthalten.

Beispiel 3

9,1 g 4,6-Dimethyltryptamin-methansulfonat werden in 250 ml Wasser gelöst und bei Raumtemperatur wird im Verlaufe von 15 Minuten eine Lösung von 3,12 g Glyoxylsäuremonohydrat in 20 ml Wasser eingetropft. Anschliessend wird das Reaktionsgemisch mit 2n-Natronlauge auf pH = 4,0 eingestellt, 30 Minuten bei Raumtemperatur gerührt und während dieser Zeit der pH dauernd nachkontrolliert bzw. nachgestellt. Anschliessend werden 7,25 g Methansulfonsäure zugefügt und das Gemisch langsam auf Rückflusstemperatur gebracht. Nach 5 Stunden Kochen bei Rückflusstemperatur wird der Inhalt des Destillationskolbens auf Raumtemperatur gebracht, mit konzentrierter Natronlauge alkalisch gestellt und mit Chloroform ausgeschüttelt. Die organische Phase wird abgetrennt, mit Wasser gewaschen und über Magnesiumsulfat getrocknet. Der erhaltene kristalline Rückstand wird aus einem Benzol-Petroläthergemisch umkristallisiert und ergibt reines 5,7-Dimethyl-2,3,4,9-tetrahydro-1H-pyrido[3,4-b]indol vom Smp. 230-232°.

Analog Beispiel 1) wird aus Base und Methansulfonsäure reines 5,7-Dimethyl-2,3,4,9-tetrahydro-1H-pyrido[3,4-b]indol-methansulfonat vom Smp. 280-282° erhalten.

Das Ausgangsmaterial kann auf folgende Weise hergestellt werden:

a) 171,0 g 2-Oxo-3-piperidincarbonsäureäthylester werden in eine Lösung von 60 g Kaliumhydroxyd in 1,8 Liter Wasser eingetragen in 12 Stunden bei 30° gerührt. Die erhaltene Lösung wird mit einer Eis-Natriumchlorid-Mischung auf 0° gekühlt und mit 1,0 n-Salzsäure auf pH = 4,5 eingestellt. Anschliessend wird eine frisch bereitete Diazoniumsalzlösung des 3,5-Dimethylanilins welche mit 2n-Natriumcarbonatlösung auf pH = 6,0 abgepuffert wurde, im Verlaufe von 10 Minuten bei 0° eingetragen.

Die Diazoniumsalzlösung wird wie folgt hergestellt:

125,5 g 3,5-Dimethylanilin werden in 500 ml Wasser und 1,2 Liter 1n-Salzsäure gelöst und das Gemisch mit Eis-Natriumchlorid-Mischung auf +10° gekühlt. Gleichzeitig lässt man eine Lösung von 74,8 g Natriumnitrit in 200 ml Wasser und 650 ml 2n-Salzsäure eintropfen und kühlt das Reaktionsgemisch fortlaufend bis auf +5° ab. Die erhaltene Lösung wird 1 Stunde bei +5° nachgerührt und ein allfälliger Nitritüberschuss mit Sulfaminsäure vernichtet. Anschliessend wird das Reaktionsgemisch, wie oben beschrieben, mit Natriumcarbonatlösung auf pH = 6,0 gepuffert.

Nach erfolgter Zugabe der Diazolösung wird das Reaktionsgemisch 2 Stunden bei +5°C gerührt, mit 40%-iger Natriumacetatlösung auf pH = 5,5 g gepuffert und 12 Stunden bei Raumtemperatur stehen gelassen. Nach erneuter Abkühlung auf 0° wird das ausgefallene Produkt abfiltriert, mit Eiswasser gewaschen und im Vakuum bei

50° getrocknet. Das erhaltene rohe 2,3-Piperidindion-3-[N'-(3,5-dimethylphenyl)hydrazon] schmilzt bei 235-240°. Eine Prope der Substanz wird mit Alkohol-Petroläther umkristallisiert und ergibt das reine 2,3-Piperidindion-3-[N'-(3,5-dimethylphenyl)-hydrazon] vom Smp. 239-241° als hellgelbe Kristalle.

b) Analog Beispiel 1b) wird aus 2,3-Piperidindion-3-[N'-(3,5-dimethylphenyl)-hydrazon] reines 5,7-Dimethyl-2,3,4,9-tetrahydro-1H-pyrido[3,4-b]indol-1-on vom Smp. 261-263° [nach Umkristallisation aus absolutem Aethylalkohol] erhalten.

c) 182,5 g 5,7-Dimethyl-2,3,4,9-tetrahydro-1H-pyridon[3,4-b]indol-1-on werden in eine Lösung von 360 g Kaliumhydroxyd in 2000 ml Wasser und 2000 ml Alkohol eingetragen und 12 Stunden am Rückfluss gekocht. Anschliessend wird der Alkohol am absteigenden Kühlen weitgehend abdestilliert und 2000 ml Wasser zugegeben. Das Reaktionsgemisch wird auf Raumtemperatur abgekühlt und mit Essigsäure auf pH = 6 eingestellt. Die erhaltene Suspension wird unter Rühren auf 0° gekühlt, das ausgefallene Produkt abfiltriert und mit wenig Eiswasser gewaschen. Eine Probe des Rohproduktes wird getrocknet, aus Dimethylformamid-Aethyläther umkristallisiert und ergibt reines 4,6-Dimethyltryptamin-2-carbonsäure-hemihydrat vom Smp. 263-265. Das feuchte oben erhaltene Rohprodukt wird ohne Reinigung in eine Mischung von 4000 ml Wasser und 400 ml konzentrierter Salzsäure eingetragen und langsam zum Rückfluss erhitzt. Das Gemisch wird 5 Stunden unter Rückfluss gekocht, auf Raumtemperatur abgekühlt und mit konzentrierter Natronlauge alkalisch gestellt. Das ausgefallene Produkt wird mit Chloroform ausgeschüttelt, die organischen Phasen mit Wasser gewaschen und

über Magnesiumsulfat getrocknet. Nach Entfernung des Lösungsmittels wird der kristalline Rückstand aus einem Toluol-Petroläther-Gemisch umkristallisiert und ergibt reines 4,6-Dimethyltryptamin vom Smp. 115-116°.

130,5 g 4,6-Dimethyltryptamin werden in 500 ml Alkohol gelöst und mit 66,5 g Methansulfonsäure versetzt. Nach Zugabe von ca. 200 ml Aether wird das ausgefallene Produkt abfiltriert und aus Alkohol-Aethyläther umkristallisiert. Es wird reines 4,6-Dimethyltryptaminmethansulfonat vom Smp. 204-205° erhalten.

Beispiel 4

14,92 g 4,5,7-Trimethyl-tryptamin-methansulfonat und 5,06 g Glyoxylsäure-monohydrat werden analog Beispiel 2 umgesetzt. Es wird rohes 5,6,8-Trimethyl-2,3,4,9-tetrahydro-1H-pyrido[3,4-b]indol erhalten. Analog Beispiel 2 wird das Methansulfonat hergestellt:

8,4 g Base und 3,76 g Methansulfonsäure ergeben nach Umkristallisation aus 90%-igem Alkohol-Aethyläther-Gemisch reines 5,6,8-Trimethyl-2,3,4,9-tetrahydro-1H-pyrido[3,4-b]indol-methansulfonat vom Smp. 290-292°.

Das Ausgangsmaterial kann auf folgende Weise hergestellt werden:

a) Analog Beispiel 3a) werden aus 25,5 g 2-Oxo-3-piperidincarbonsäure-äthylester und 21,0 g 2,4,5-Trimethylanilin 20,8 g 2,3-Piperidindion-3-[N'-(2,4,5-trimethylphenyl)-hydrazon] vom Smp. 198-200° erhalten.

b) Analog Beispiel 1b) wird aus 2,3-Piperidin-dion-3-[(2,4,5-trimethylphenyl)-hydrazon] reines 5,6,8-Trimethyl-2,3,4,9-tetrahydro-1H-pyrido[3,4-b]indol-1-on vom Smp. 213-215° erhalten.

c) Analog Beispiel 3c) wird aus 5,6,8-Trimethyl-2,3,4,9-tetrahydro-1H-pyrido[3,4-b]indol-1-on 4,5,7-Trimethyl-tryptamin vom Smp. 169-171° erhalten. Aus der freien Base wird analog Beispiel 3c) mit Methansulfonsäure des 4,5,7-Trimethyltryptamin-methansulfonat vom Smp. 274-276 hergestellt.

Beispiel 5

Analog Beispiel 3) wird aus 5,7-Dimethyltryptamin-methansulfonat und Glyoxylsäure-monohydrat 6,8-Dimethyl-2,3,4,9-tetrahydro-1H-pyrido[3,4-b]indol vom Smp. 270-272° erhalten. Aus der freien Base wird analog Beispiel 3 6,8-Dimethyl-2,3,4,9-tetrahydro-1H-pyrido[3,4-b]indol-methansulfonat vom Smp. 286-288° erhalten.

Das Ausgangsmaterial kann auf folgende Weise hergestellt werden:

a) Analog Beispiel 3a) wird aus 2,4 Dimethylanilin und 2-Oxo-3-piperidincarbonsäureäthylester-2,3-Piperidin-dion-3-[(2,4-dimethylphenyl)-hydrazon] vom Smp. 187-189° erhalten.

b) Analog Beispiel 1b) wird aus 2,3-Piperidin-dion-3-[(2,4-dimethylphenyl)-hydrazon] 6,8-Dimethyl-2,3,4,9-tetrahydro-1H-pyrido[3,4-b]indol-1-on vom Smp. 217-221° erhalten.

c) Analog Beispiel 3c) wird aus 6,8-Dimethyl-2,3,4,9-tetrahydro-1H-pyrido-[3,4-b]indol-1-on 5,7-Dimethyltryptamin vom Smp. 112-114° erhalten. Das Methansulfonat schmilzt bei 228-230°.

Beispiel 6

Analog Beispiel 3 wird aus 4,6-Dimethyl-5-methoxy-tryptamin-methansulfonat und Glyoxylsäure-monohydrat das 5,7-Dimethyl-6-methoxy-2,3,4,9-tetrahydro-1H-pyrido[3,4-b]indol vom Smp. 236-240° erhalten.

Aus der freien Base wird analog Beispiel 3 das 5,7-Dimethyl-6-methoxy-2,3,4,9-tetrahydro-1H-pyrido [3,4-b]indol-methansulfonat vom Smp. 262-265° erhalten.

Das Ausgangsmaterial kann auf folgende Weise hergestellt werden:

a) Analog Beispiel 3a) wird aus 3,5-Dimethyl-4-methoxyanilin (Baldwin u. Robinson J.chem.Soc. 1934 1264, 1266) und 2-Oxo-3-piperidincarbonsäureäthylester 2,3-Piperidindion-3-[N'-(3,5-dimethyl-4-methoxy-phenyl)-hydrazon] vom Smp. 236 - 238° erhalten.

b) Analog Beispiel 1b) wird aus 2,3-Piperidindion-3[(3,5-dimethyl-4-methoxy-phenyl)-hydrazon] reines 5,7-Dimethyl-4-methoxy-2,3,4,9-tetrahydro-1H-pyrido [3,4-b]indol-1-on vom Smp. 235-237° erhalten.

c) Analog Beispiel 3c) wird aus 5,7-Dimethyl-6-methoxy-2,3,4,9-tetrahydro-1H-pyrido[3,4-b]indol-1-on das 4,6-Dimethyl-5-methoxy-tryptamin in Form eines Oeles erhalten.

Aus der freien Base wird analog Beispiel 3c) das 4,6-Dimethyl-5-methoxy-tryptamin-methansulfonat vom Smp. 251 - 253° hergestellt.

Beispiel 7

21,4 g 5,7-Dimethyl-2,3,4,9-tetrahydro-1H-pyrido[3,4-b]indol-1-on werden in den oberen Teil eines Extraktors [Soxhletapparat mit Glasfritte] gefüllt. In den Rückflusskolben werden 7,56 g Lithiumaluminiumhydrid in 1000 ml Aethyläther gegeben. Der Aether wird unter Stickstoffatmosphäre ca. 74 Stunden bis zur vollständigen Lösung der eingetragenen Substanz am Rückfluss gehalten. Anschliessend wird der Kolben mit Eis gekühlt und das überschüssige Lithiumaluminiumhydrid durch Zugabe von 1.) 7,5 ml Wasser, 2.) 7,5 ml 15%-ige Natronlauge und 3.) 22,5 ml Wasser zerstört.

Der ausgefallene Niederschlag wird abfiltriert und 4 mal mit 500 ml Toluol ausgekocht. Die Toluolauszüge werden mit der als Filtrat erhaltenen Aetherlösung vereinigt und mit 1-molarer Methansulfonsäure extrahiert. Die saure wässerige Lösung wird mit konzentrierter Natronlauge alkalisch gestellt und mit Methylenchlorid ausgezogen. Die organische Phase wird mit Wasser gewaschen, über Magnesiumsulfat getrocknet und das Lösungsmittel unter Vakuum entfernt. Der erhaltene kristalline Rückstand wird aus einem Benzol-Petroläther-Gemisch umkristallisiert und ergibt reines 5,7-Dimethyl-2,3,4,9-tetrahydro-1H-pyrido[3,4-b]indol vom Smp. 230-232°.

13,0 g 5,7-Dimethyl-2,3,4,9-tetrahydro-1H-pyrido-[3,4-b]indol werden in 100 ml absolutem Aethanol gelöst und mit 6,25 g Methansulfonsäure versetzt. Nach Zugabe von 50 ml Aethyläther wird das ausgefallene Produkt abfiltriert und aus 96%-igem Aethanol umkristallisiert. Es wird reines 5,7-Dimethyl-2,3,4,9-tetrahydro-1H-pyrido[3,4-b]indol-methansulfonat vom Smp. 280 - 282° erhalten.

Beispiel 8

Analog Beispiel 7 werden erhalten:

aus 6,8-Dimethyl-2,3,4,9-tetrahydro-1H-pyrido[3,4-b] indol-1-on das 6,8-Dimethyl-2,3,4,9-tetrahydro-1H-pyrido[3,4-b]indol vom Smp. 270-272° und sein Methansulfonat vom Smp. 286-288°, und

aus 5,6,8-Trimethyl-2,3,4,9-tetrahydro-1H-pyrido[3,4-b] indol-1-on das 5,6,8-Trimethyl-2,3,4,9-tetrahydro-1H-pyrido[3,4-b]indol-methansulfonat vom Smp. 290-292°.

Beispiel 9

2,0 g 5,7-Dimethyl-2,3,4,9-tetrahydro-1H-pyrido [3,4-b]indol und 1,81 g 1 Bromhexan werden mit 1,42 g Kaliumcarbonat in 50 ml Aceton eingetragen und unter Rühren 36 Stunden unter Rückfluss gekocht. Das Reaktionsgemisch wird etwas abgekühlt, durch Kieselgur filtriert und der Rückstand mit heissem Aceton ausgezogen. Die vereinigten Filtrate werden am Vakuum eingedampft und der verbliebene Rückstand mit Toluol und Wasser geschüttelt. Die organische Phase wird mit Wasser gewaschen und mit Magnesiumsulfat getrocknet. Nach Entfernen des Lösungsmittels wird der kristalline Rückstand 1 mal aus einem Toluol-Petroläther-Gemisch umkristallisiert und ergibt reines 2-n—Hexyl-5,7-dimethyl-2,3,4,9-tetrahydro-1H-pyrido[3,4-b]indol vom Smp. 124-126°.

Beispiel 10

Analog Beispiel 9 wird aus 5,7-Dimethyl-2,3,4,9-tetrahydro-1H-pyrido[3,4-b]indol und p-Fluor-benzylbromid das 2-(p-Fluorbenzyl)-5,7-dimethyl-2,3,4,9-tetrahydro-1H-

pyrido[3,4-b]indol vom Smp. 168-170° erhalten.

**Beispiel 11**

Analog Beispiel 9 wird mit Benzylbromid das 2-Benzyl-5,7-dimethyl-2,3,4,9-tetrahydro-1H-pyrido [3,4-b]indol vom Smp. 157-159° erhalten.

**Beispiel 12**

2,0 g 5,7-Dimethyl-2,3,4,9-tetrahydro-1H-pyrido [3,4-b]indol werden mit 1,2 g Benzaldehyd in 50 ml Benzol 4 Stunden am Rückfluss gehalten. Anschliessend wird das Benzol weitgehend abdestilliert und der Rückstand in 50 ml Methanol aufgenommen und bei Rückflusstemperatur eine Lösung von 0,57 g Natriumborhydrid in 10 ml Eiswasser eingetropft. Das Gemisch wird 3 Stunden unter Rückfluss gehalten, abgekühlt, mit 50 ml Wasser versetzt und mit 2n.-Salzsäure sauer gestellt. Anschliessend wird das Methanol abdestilliert und die erhaltene Lösung mit 2n-Natronlauge alkalisch gestellt und mit Methylenchlorid ausgezogen. Die organischen Phasen werden mit Wasser gewaschen, über Magnesiumsulfat getrocknet und das Lösungsmittel am Vakuum entfernt. Der Rückstand wird aus einem Aethyläther-Pentan-Gemisch kristallisiert und ergibt das 2-Benzyl-5,7-dimethyl-2,3,4,9-tetrahydro-1H-pyrido[3,4-b]indol vom Smp. 158-160°.

**Beispiel 13**

12,8 g 2-Acetyl-5,7,9-trimethyl-2,3,4,9-tetrahydro-1H-pyrido[3,4-b]indol werden mit 250 ml 1,5n-Natronlauge und 250 ml Aethanol 5 Stunden am Rückfluss gekocht. Anschliessend wird der Aethanol am Vakuum entfernt und die erhaltene Suspension mit Methylenchlorid ausgeschüttelt. Die organische Phase wird mit Wasser gewaschen, über Magnesiumsulfat getrocknet und das Lösungsmittel am Vakuum entfernt. Der erhaltene Rückstand wird aus Aethylacetat umkristallisiert und ergibt reines 5,7,9-Trimethyl-2,3,4,9-tetrahydro-1H-pyrido [3,4-b]indol vom Smp. 140-142°.

Die erhaltene Base wird in 30 ml absolutem Aethanol gelöst, mit 3,8 g Methansulfonsäure versetzt und 30 ml Aethyläther zugegeben. Das ausgefallene Produkt wird abfiltriert und aus absolutem Aethanol-Aethyläther-Gemisch umkristallisiert. Es wird reines 5,7,9-Trimethyl-2,3,4,9-tetrahydro-1H-pyrido[3,4-b]indol-methansulfonat vom Smp. 263-264° erhalten.

Das Ausgangsmaterial kann auf folgende eise hergestellt werden:

a) 30 g 5,7-Dimethyl-2,3,4,9-tetrahydro-1H-pyrido[3,4-b]indol werden mit 12,3 g Natriumacetat in 500 ml Acetanhydrid 4 Stunden auf 70° erwärmt. Anschliessend wird das Acetanhydrid unter Vakuum weitgehend entfernt und der feste Rückstand auf Wasser gegossen und während 30 Minuten auf 50° erwärmt. Die erhaltene Suspension wird mit Methylenchlorid ausgeschüttelt, die organische Phase mit 1n-Natriumhydrogencarbonatlösung und mit Wasser gewaschen und über Magnesiumsulfat getrocknet. Nach Entfernen des Lösungsmittels unter Vakuum wird der erhaltene kristalline Rückstand aus einem Aethylacetat-Petroläther-Gemisch umkristallisiert. Es wird reines 2-Acetyl-5,7-dimethyl-2,3,4,9-tetrahydro-1H-pyrido[3,4-b]indol vom Smp. 190-192° erhalten.

b) 24,2 g 2-Acetyl-5,7-dimethyl-2,3,4,9-tetrahydro-1H-pyrido[3,4-b]indol werden in 500 ml Methylenchlorid gelöst und bei Raumtemperatur mit einer Lösung von 16,9 g Tetrabutylammoniumhydrogensulfat in 250 ml 50%-iger Natronlauge versetzt. Das Gemisch wird mit einem Vibromischer während ca. 3 Minuten kräftig vibriert, sodass eine dickflüssige Emulsion entsteht. Anschliessend werden unter weiterer guter Durchmischung 13,8 g Dimethylsulfat in 50 ml Methylenchlorid bei max. 25° eingetropft (kühlen mit Eisbad), und das Gemisch 1 Stunde bei Raumtemperatur vibriert. Anschliessend wird der Kolbeninhalt auf Eis gegossen, das Methylenchlorid abgetrennt und die wässerige Phase 2 mal mit 200 ml Methylenchlorid ausgeschüttelt. Die vereinigten wässerigen Phasen werden mit Wasser gewaschen und über Magnesiumsulfat getrocknet. Nach Entfernung des Lösungsmittels unter Vakuum wird der Rückstand aus einem Aethylacetat-Petroläther-Gemisch umkristallisiert. Es wird reines 2-Acetyl-5,7,9-trimethyl-2,3,4,9-tetrahydro-1H-pyrido[3,4-b]indol vom Smp. 150-151° erhalten.

## Beispiel 14

Analog Beispiel 13) wird aus 2-Acetyl-6,8,9-trimethyl-2,3,4,9-tetrahydro-1H-pyrido[3,4-b]indol das 6,8,9-Trimethyl-2,3,4,9-tetrahydro-1H-pyrido[3,4-b]indol vom Smp. 186-188° erhalten.

Das Ausgangsmaterial kann auf folgende Weise hergestellt werden:

a) Analog Beispiel 13a) wird aus 6,8-Dimethyl-2,3,4,9-tetrahydro-1H-pyrido[3,4-b]indol das 2-Acetyl-6,8-dimethyl-2,3,4,9-tetrahydro-1H-pyrido[3,4-b]indol vom Smp. 192-195° erhalten.

b) Analog Beispiel 13b) wird aus 2-Acetyl-6,8-dimethyl-2,3,4,9-tetrahydro-1H-pyrido[3,4-b]indol das 2-Acetyl-6,8,9-trimethyl-2,3,4,9-tetrahydro-1H-pyrido [3,4-b]indol vom Smp. 129-130° erhalten.

Beispiel 15

Analog Beispiel 9) wird aus 5,7,9-Trimethyl-2,3,4,9-tetrahydro-1H-pyrido[3,4-b]indol und 2-Propinylbromid das 2-(2-Propinyl)-5,7,9-trimethyl-2,3,4,9-tetrahydro-1H-pyrido[3,4-b]indol vom Smp. 160-161° erhalten. Das Hydrochlorid, hergestellt mit Salzsäure in Aethanol, schmilzt bei 233-234°.

Beispiel 16

Analog Beispiel 9) wird aus 5,7,9-Trimethyl-2,3,4,9-tetrahydro-1H-pyrido[3,4-b]indol und Benzylbromid das 2-Benzyl-5,7,9-trimethyl-2,3,4,9-tetrahydro-1H-pyrido[3,4-b]indol erhalten; Smp. 135-137°. Das Maleat schmilzt bei 178-180° (aus absolutem Aethanol-Aether).

Beispiel 17

Analog Beispiel 9) wird aus 5,7,9-Trimethyl-2,3,4,9-tetrahydro-1H-pyrido[3,4-b]indol und p-Fluorbenzylbromid das 2-(p-Fluorbenzyl)-5,7,9-trimethyl-2,3,4,9-tetrahydro-1H-pyrido[3,4-b]indol erhalten; Smp. 152-154°.

16,1 g Base werden in 50 ml Aceton gelöst und mit 5,8 g Maleinsäure versetzt. Nach Zugabe von 50 ml Aethylacetat wird das ausgefallene Produkt abfiltriert und aus 90%-igem Aethanol-Aether-Gemisch umkristallisiert. Es

wird reines 2-(p-Fluorbenzyl)-5,7,9-trimethyl-2,3,4,9-tetrahydro- 1H -pyrido[3,4-b]indol-maleat vom Smp. 185-187° erhalten.

Beispiel 18

Analog Beispiel 9) wird aus 5,7,9-Trimethyl-2,3,4,9-tetrahydro-1H-pyrido[3,4-b]indol und 2-Bromäthanol das 2-[2-Hydroxyäthyl)-5,7,9-trimethyl-2,3,4,9-tetrahydro-1H-pyrido[3,4-b]indol erhalten; Smp. 140 - 141°.

Aus der freien Base und äthanolischer Salzsäure wird nach Umkristallisation aus einem Alkohol-Aether-Gemisch das reine 2-(2-Hydroxyäthyl)-5,7,9-trimethyl-2,3,4,9-tetrahydro-1H-pyrido[3,4-b]indol-hydrochlorid vom Smp. 253-255° erhalten.

Beispiel 19

24,2 g 2-Formyl-5,7,9-trimethyl-2,3,4,9-tetrahydro-1H-pyrido[3,4-b]indol werden in kleinen Portionen zu einer kochenden Lösung von 3,8 g Lithiumaluminiumhydrid in 500 ml Aethyläther gegeben. Das Reaktionsgemisch wird 12 Stunden am Rückfluss gehalten und anschliessend unter Eiskühlung das überschüssige Lithiumaluminiumhydrid durch Zugabe von 4 ml Wasser, 4 ml 15%-iger Natronlauge und 11 ml Wasser zerstört. Der Aether wird vom ausgefallenen körnigen Niederschlag abfiltriert und diesen 3 mal mit heissem Toluol ausgekocht. Die organischen Filtrate werden vereinigt, über Magnesiumsulfat getrocknet und die Lösungsmittel unter Vakuum entfernt. Das erhaltene kristalline Rohprodukt wird aus einem Aethyläther-Pentan-Gemisch umkristallisiert und ergibt reines 2,5,7,9-Tetramethyl-2,3,4,9-tetra-

hydro-1H-pyrido[3,4-b]indol vom Smp. 144-145°.

Das Ausgangsmaterial kann wie folgt hergestellt werden:

21,4 g 5,7,9-Trimethyl-2,3,4,9-tetrahydro-1H-pyrido[3,4-b]indol werden in 500 ml Ameisensäureäthylester gelöst und 12 Stunden unter Rückfluss gekocht. Der überschüssige Ameisensäureäthylester wird unter Vakuum entfernt und der kristalline Rückstand aus einem Aethylalkohol-Petroläther-Gemisch umkristallisiert. Es wird reines 2-Formyl-5,7,9-trimethyl-2,3,4,9-tetrahydro-1H-pyrido[3,4-b]indol vom Smp. 158-160° erhalten.

Beispiel 20

6,4 g 5,7,9-Trimethyl-2,3,4,9-tetrahydro-1H-pyrido[3,4-b]indol-1-on werden in einen Glasfrittenextraktor gebracht und in eine siedende Lösung von 2,4 g Lithiumaluminiumhydrid in 400 ml abs. Aether extrahiert. Der Kolbeninhalt wird mit einem Vribomischer stark bewegt. Nach ca. 30 Stunden ist die Extraktion beendigt, der Kolben wird mit Eis gekühlt und nacheinander werden je 2,2 ml Wasser, 2,2 ml 15%-ige Natronlauge und 6,6 ml Wasser eingetropft. Der erhaltene körnige Niederschlag wird über Kieselgur abfiltriert, 3mal mit je 100 ml Methylenchlorid ausgekocht und erneut abfiltriert. Die vereinigten Filtrate werden über Magnesiumsulfat getrocknet und am Vakuum zur Trockne eingedampft. Der erhaltene Rückstand wird aus Toluol-Petroläther umkristallisiert und ergibt 5,0 g 5,7,9-Trimethyl-2,3,4,9-tetrahydro-1H-pyrido[3,4-b]indol vom Smp. 142-143°.

Das Ausgangsmaterial kann wie folgt hergestellt werden:

a) 87 g 3,5-Dimethylanilin werden in 1570 ml 1n-Salzsäure gelöst, auf -3° abgekühlt und bei dieser Temperatur eine Lösung von 49,6 g Natriumnitrit in 142 ml Wasser im Verlaufe von 15 Minuten eingetropft. Die klare hellgelbe Lösung wird 5 Minuten bei -2° gerührt und ein allfälliger Ueberschuss an Nitrit mit einer 1-molaren Sulfaminsäurelösung vernichtet [Probe mit Kaliumjodid-Stärke-Papier]. Anschliessend wird bei -2° bis 0° eine Lösung von 102 g 2-Oxo-3-piperidincarbonsäure-äthylester in 300 ml Wasser rasch zugegeben und im Verlaufe von 50 Minuten ca. 800 ml einer halbgesättigten Kaliumcarbonatlösung versetzt, so dass der pH 9 erreicht wird. Nach der Zugabe von ca. 30 ml dieser Lösung beginnt ein brauner schmieriger Niederschlag auszufallen. Er wird durch Zugabe von 100 ml Toluol-Hexan 3:7 in kristalliner Form erhalten. Das Reaktionsgemisch wird 1 Stunde bei 0° nachgerührt, der ausgefallene Niederschlag abfiltriert, mit kaltem Wasser neutral gewaschen und über Nacht im Vakuumexsiccator getrocknet. Es werden gelbe Kristalle von Smp. 91-96° als Rohprodukt erhalten (noch etwas feucht). Das reine 4-Aethoxycarbonyl-2,3-piperidindion-3-[N'-(3,5-dimethylphenyl)hydrazon] schmilzt nach Umkristallisation aus Toluol-Petroläther bei 106-109°.

b) 204,5 g des oben erhaltenen Rohproduktes werden in 1200 ml Methanol gelöst, über Kieselgur filtriert und die erhaltene klare Lösung auf 8° abgekühlt. Anschliessend werden 600 ml 1n-Natronlauge zugegeben und das Gemisch 2 Stunden bei Raumtemperatur gerührt. Anschliessend wird das Gemisch auf 0° gekühlt, mit 2n-Salzsäure auf pH 6-7 gestellt, und nach 1 Stunde Rühren werden im Eisbad die ausgefallenen Kristalle abfiltriert. Der Rückstand wird

vorerst mit 200 ml eiskaltem Methanol-Wasser-Gemisch 1:1 und anschliessend mit Wasser nachgewaschen. Die erhaltenen Kristalle werden im Vakuum getrocknet und anschliessend mit Aether ausgewaschen. Das erhaltene Rohprodukt schmilzt bei 217°. Eine Probe des Rohproduktes wird aus Alkohol-Aether umkristallisiert und ergibt das reine 2,3-Piperidindion-3-[N'-(3,5-dimethylphenyl)hydrazon] vom Smp. 239-240° als hellgelbe Kristalle.

c) Das 5,7-Dimethyl-2,3,4,9-tetrahydro-1H-pyridon[3,4-b]indol-1-on kann gemäss Beispiel 3 b) hergestellt werden.

d) In einen Dreihals-Reaktionskolben werden 80 ml 50%-ige Natronlauge und 6,76 g Tetrabutylammoniumhydrogensulfat gegeben. Anschliessend wird eine Lösung von 8,56 g 5,7-Dimethyl-2,3,4,9-tetrahydro-1H-pyridon[3,4-b]indol-1-on in 200 ml Methylenchlorid beigefügt. Das Reaktionsgemisch wird mit dem Vibromischer stark vermischt, und bei 20° wird im Verlaufe von 20 Minuten eine Lösung von 5,52 g Dimethylsulfat in 20 ml Methylenchlorid eingetropft. Nach Beendigung der Zugabe wird das Reaktionsgemisch weitere 30 Minuten vibriert. Anschliessend werden 100 ml Wasser beigefügt, die Methylenchloridphase abgetrennt und das Wasser je 3mal mit 100 ml Methylenchlorid extrahiert. Die organischen Auszüge werden je 3mal mit 100 ml Wasser ausgewaschen und vereinigt. Die organischen Phasen werden über Magnesiumsulfat getrocknet und am Vakuum zur Trockne eingedampft. Der erhaltene Rückstand wird mit Aethylacetat ausgekocht und der gelöste Anteil aus wenig Aethylacetat-Petroläther kristallisiert. Es wird reines 5,7,9-Trimethyl-2,3,4,9-tetrahydro-1H-pyrido[3,4-b]indol-1-on vom Smp. 237-238° erhalten.

Beispiel 21

4,2 g 5,7,9-Trimethyl-3,4-dihydro-9H-pyrido[3,4-b]indol werden in 200 ml absolutem Methanol gelöst und eine Lösung von 1,92 g Methansulfonsäure in 20 ml Methanol wird zugefügt. Anschliessend werden bei Raumtemperatur 1,24 g Natriumcyanoborhydrid, gelöst in 20 ml absolutem Methanol, zugefügt und im Verlaufe von ca. 8 Stunden eine Lösung von 1,92 g Methansulfonsäure, gelöst in 30 ml absolutem Methanol, zugetropft, so dass der pH des Reaktionsgemisches immer zwischen 5-6 liegt. Das Gemisch wird 48 Stunden bei Raumtemperatur stehen gelassen, mit 1 mol Methansulfonsäure angesäuert (pH$\sim$1) und das Methanol im Vakuum entfernt. Der erhaltene Rückstand wird 2mal mit je 50 ml Methanol eingedampft, in 100 ml Wasser gelöst und dann mit 2n-Natronlauge basisch gestellt. Das Gemisch wird 3mal mit je 50 ml Methylenchlorid ausgeschüttelt, die organischen Phasen abgetrennt und über Magnesiumsulfat getrocknet. Nach dem Entfernen des Lösungsmittels wird der erhaltene Rückstand aus Toluol-Petroläther umkristallisiert. Es wird reines 5,7,9-Trimethyl-2,3,4,9-tetrahydro-1H-pyrido[3,4-b]indol vom Smp. 140° erhalten.

Das Ausgangsmaterial kann wie folgt hergestellt werden:

a) 11,45 g 5,7,9-Trimethyl-2,3,4,9-tetrahydro-1H-pyrido[3,4-b]indol-1-on werden zu einer Lösung von 21,7 g Kaliumhydroxyd in 150 ml Wasser und 150 ml Alkohol (7%-ige Lösung) suspendiert und das Gemisch 48 Stunden am Rückfluss gehalten. Die fast klare Lösung wird abgekühlt, über Kieselgur filtriert und das Filtrat mit Essigsäure auf pH = 6 angesäuert. Nach Abkühlen mit Eis wird das ausgefallene Produkt abfiltriert, mit Wasser gewaschen und im Vakuum bei 60° getrocknet. Die erhaltene 1,4,6-Trimethyl-tryptamin-2-carbonsäure [Fp: 233-235°] wird roh weiterverwendet.

b) 7,8 g 1,4,6-Trimethyltryptamin-2-carbonsäure werden in 300 ml 5%-iger Salzsäure suspendiert und unter Rückfluss erhitzt. Nach 5 Stunden wird eine klare Lösung erhalten. Die Lösung wird abgekühlt, mit konz. Natronlauge alkalisch gestellt, und mit Methylenchlorid ausgeschüttelt. Die organische Phase wird mit Wasser gewaschen, über Magnesiumsulfat getrocknet und das Lösungsmittel am Vakuum entfernt. Der erhaltene kristalline Rückstand wird aus Pentan umkristallisiert und ergibt reines 1,4,6-Trimethyltryptamin vom Smp. 50-51°.

c) 5,45 g 1,4,6-Trimethyltryptamin werden in 40 ml Ameisensäureäthylester suspendiert und im Bombenrohr 2 Stunden auf 100° erwärmt. Die erhaltene klare Lösung wird im Vakuum zur Trockne eingedampft und der Rückstand aus Toluol-Petroläther umkristallisiert. Es wird reines N-Formyl-1,4,6-trimethyltryptamin vom Smp. 125-126° erhalten.

d) 4,2 g N-Formyl-1,4,6-trimethyltryptamin werden in 600 ml Toluol gelöst und unter gutem Rühren 47 g Phosphorpentoxyd in kleinen Portionen im Verlaufe von 30 Minuten eingetragen. Das Gemisch wird für 15 Minuten am Rückfluss erwärmt, abgekühlt und auf ca. 1 l Eiswasser gegossen. Das erhaltene Gemisch wird mit ca. 50 ml 2n-Salzsäure versetzt, auf Raumtemperatur gebracht und 1 Stunde bei Raumtemperatur gerührt. Die wässerige gelbe Lösung wird abgetrennt, mit konz. Natronlauge alkalisch gestellt und mit Methylenchlorid ausgeschüttelt. Die organischen Extrakte werden mit Wasser gewaschen, über Magnesiumsulfat getrocknet und das Lösungsmittel im Vakuum entfernt. Der erhaltene Rückstand wird mit Aether verrieben, die kristallinen Anteile abfiltriert und im Vakuum bei 40° getrocknet. Es

wird reines 5,7,9-Trimethyl-3,4-dihydro-9H-pyrido[3,4-b]indol vom Smp. 181-185° erhalten.

Beispiel 22

2,1 g 5,7,9-Trimethyl-9H-pyrido[3,4-b]indol werden in 100 ml absolutem Aethanol gelöst und unter Zusatz von 0,96 g Methansulfonsäure und 1 g 5%-iger Palladiumkohle bei 40-50° und 5 bar Druck katalytisch hydriert. Nach Beendigung der Wasserstoffaufnahme wird vom Katalysator abfiltriert und das Filtrat zur Trockne eingedampft. Der Rückstand wird in Wasser gelöst, mit 2n-Natronlauge alkalisch gestellt und mit Methylenchlorid ausgeschüttelt. Die organische Phase wird mit Wasser gewaschen, über Magnesiumsulfat getrocknet und das Lösungsmittel im Vakuum entfernt. Der erhaltene kristalline Rückstand wird aus Toluol-Petroläther umkristallisiert. Es wird reines 5,7,9-Trimethyl-2,3,4,9-tetrahydro-1H-pyrido[3,4-b]indol vom Smp. 140-142° erhalten.

Das Ausgangsmaterial kann wie folgt erhalten werden:

3,44 g 5,7,9-Trimethyl-2,3,4,9-tetrahydro-1H-pyrido[3,4-b]indol-1-on werden in 40 ml Phosphoroxychlorid gelöst und 4 Stunden unter Rückfluss gehalten. Anschliessend wird das Gemisch am Vakuum zur Trockne eingedampft und der kristalline Rückstand in Wasser gelöst und mit Natronlauge alkalisch gestellt. Das erhaltene Gemisch wird mit Methylenchlorid ausgeschüttelt und die organische Phase mit Wasser gewaschen. Die erhaltenen Methylenchloridextrakte werden mit 1 mol Zitronensäure ausgeschüttelt, die saure wässerige Phase abgetrennt und mit 2n-Natronlauge alkalisch gestellt. Die alkalische wässerige Phase wird wiederum mit Methylenchlorid ausgeschüttelt. Die organischen Phasen werden mit Wasser gewaschen, über Magnesiumsulfat getrocknet und das Lösungsmittel im Vakuum entfernt. Der erhaltene kristalline

Rückstand wird aus Toluol-Petroläther umkristallisiert. Es wird reines kristallines 5,7,9-Trimethyl-9H-pyrido[3,4-b] indol vom Smp. 151-152° erhalten.

Beispiel 23

21,4 g 5,7,9-Trimethyl-2,3,4,9-tetrahydro-1H-pyrido [3,4-b]indol werden mit 12,9 g Diisopropylamin und 14,4 g Chlormethylcyclopropan in 200 ml Alkohol 24 Stunden am Rückfluss gekocht. Zur Aufarbeitung wird der Alkohol am Vakuum entfernt und der erhaltene Rückstand mit Methylenchlorid und 2n-Ammoniak ausgeschüttelt. Die organischen Phasen werden mit Wasser gewaschen, abgetrennt und über Magnesiumsulfat getrocknet. Nach Entfernen des Methylenchlorids am Vakuum wird der erhaltene ölige Rückstand über eine Säule mit 100 g basischem Aluminiumoxyd filtriert. (Elutionsmittel: Toluol + 5% Methanol). Die Eluate werden am Vakuum zur Trockene eingedämpft und der erhaltene ölige Rückstand in Essigsäureäthylester mit ätherischer Salzsäure bis zur sauren Reaktion versetzt. Das ausgefallene Hydrochlorid wird aus 80%-igem Alkohol und Aether umkristallisiert und ergibt das reine 2-Cyclopropyl-methyl-5,7,9-trimethyl-2,3,4,9-tetrahydro-1H-pyrido[3,4-b]indol-hydrochlorid vom Smp. 275-278°.

Beispiel 24

29,8 g 1,4,6-Trimethyl-tryptamin-methansulfonat werden in 300 ml Wasser gelöst und bei Raumtemperatur eine Lösung von 9,7 g Brenztraubensäure in 60 ml Wasser eingetropft. Anschliessend werden ca. 120 ml 1n-Kalilauge ebenfalls bei Raumtemperatur langsam eingetropft, sodass ein End-pH-Wert von 3,8-4,0 erhalten wird. Das erhaltene Gemisch wird 5 Stunden bei Raumtemperatur gerührt und nötigenfalls der pH-Wert nachgestellt. Die erhaltene Suspension wird langsam

und in kleinen Portionen zu einem auf 100° erhitzten Gemisch von 300 ml Wasser und 29 g Methanolsulfosäure gegeben. Das Reaktionsgemisch wird kräftig gerührt da starkes Aufschäumen stattfindet. Nach beendigter Zugabe wird die Suspension 4 Stunden am Rückfluss gehalten bis eine klare gelbe Lösung resultiert. Zur Aufarbeitung werden in die warme Lösung ca. 40 ml konz. Natronlauge eingetragen (stark alkalische Reaktion) und nach erfolgter Abkühlung mit Methylenchlorid ausgeschüttelt. Die organischen Phasen werden 3mal mit Wasser gewaschen, über Magnesiumsulfat getrocknet und am Vakuum zur Trockene eingedampft. Der erhaltene ölige Rückstand wird durch eine Säule mit 50 g basischen Silicagel filtriert (Toluol + 2% Methanol) und die Eluate zur Trockene eingedampft. Der erhaltene Rückstand wird aus Petroläther/ Pentan kristallisiert und ergibt das reine 1,5,7,9-Tetramethyl-2,3,4,9-tetrahydro-1H-pyrido[3,4-b]indol vom Smp. 86 –87°.

14,0 g 1,5,7,9-Tetramethyl-2,3,4,9-tetrahydro-1H-pyrido[3,4-b]indol werden in 30 ml Aceton gelöst und mit einer Lösung von 7,14 g Maleinsäure in 30 ml Aceton versetzt. Nach Zugabe von Aether bis zu Trübung wird das Gemisch auf 0° abgekühlt und das ausgefallene Produkt abfiltriert. Nach Umkristallation aus abs. Alkohol-Aethyläther wird das reine 1,5,7,9-Tetramethyl-2,3,4,9-tetrahydro-1H-pyrido[3,4-b]indol-maleat vom Smp. 194 –196° erhalten.

Analog wird aus 29 g 4,6 Dimethyl-tryptamin-methansulfonat und 10 g Brenztraubensäure reines 1,5,7-Trimethyl-2,3,4,9-tetrahydro-1H-pyrido[3,4-b]indol erhalten.

Analog wird aus 2,3 g 1,4,6-Trimethyl-tryptamin-methansulfonat und 1,02 g 2-Oxobuttersäure das 1-Aethyl-5,7,9-trimethyl-2,3,4,9-tetrahydro-1H-pyrido[3,4-b]indol erhalten.

Beispiel 25

6,5 g 1,4,6-Trimethyltryptamin-methansulfonat werden in 50 ml Wasser und 50 ml Alkohol gelöst und bei 5° 1,8 g Isobutyraldehyd eingetropft. Das Gemisch wird mit 1n-Natronlauge auf pH 4,5 gestellt und 5 Stunden bei 35° gerührt. Anschliessend wird das Gemisch mit Natronlauge alkalisch gestellt und mit Methylenchlorid ausgeschüttelt. Die organischen Extrakte werden mit 1 molarer (50 ml) Methansulfosäure ausgeschüttelt, die sauren wässerigen Phasen erneut mit Natronlauge alkalisch gestellt und mit Methylenchlorid ausgezogen. Die organischen Phasen werden mit Wasser ausgewaschen über Magnesiumsulfat getrocknet und das Lösungsmittel am Vakuum entfernt. Der Rückstand wird aus Aether-Pentan umkristallisiert und ergibt das 1-Isopropyl-5,7,9-trimethyl-2,3,4,9-tetrahydro-1H-pyrido[3,4-b]indol.

Beispiel 26

Analog Beispiel 21 wird aus 2,26 g 1,5,7,9-Tetramethyl-3,4-dihydro-9H-pyrido[3,4-b]indol, das 1,5,7,9-Tetramethyl-2,3,4,9-tetrahydro-1H-pyrido[3,4-b]indol vom Smp. 86–87° erhalten.

Das Ausgangsmaterial kann wie folgt erhalten werden:

a) 10,1 g 1,4,6-Trimethyl-tryptamin werden mit 41 g Natriumacetat in 150 ml Acetanhydrid 4 Stunden auf 70° erwärmt. Das überschüssige Acetanhydrid wird am Vakuum weitgehend entfernt und der Rückstand auf 200 ml Wasser gegossen. Nach vollständiger Hydrolyse des Acetanhydrids wird die erhaltene Suspension mit Methylenchlorid ausgezogen. Die organischen Phasen werden mit Magnesiumsulfat getrocknet und am Vakuum zur Trockene eingedampft.

b) Das so erhaltene rohe N-Acetyl-1,4,6-trimethyl-tryptamin wird mit 80 g Polyphosphorsäureäthylester (nach Fiesen und Fiesen, Org.Reagents, Vol I,S. 892) unter Rühren 15 Minuten auf 80° erwärmt. Das Reaktionsgemisch wird anschliessend auf 2 Liter Eiswasser gegossen und 3 Stunden bei Raumtemperatur gerührt. Die erhaltene Suspension wird filtriert, die Filtrate mit konz. Natronlauge alkalisch gestellt und mit Methylenchlorid ausgeschüttelt. Die organischen Extrakte werden mit Wasser gewaschen,über Magnesiumsulfat getrocknet und am Vakuum zur Trockene eingedampft. Der Rückstand wird aus 10%-igem Alkohol umkristallisiert und ergibt das reine 1,5,7,9-Tetramethyl-3,4-dihydro-9H-pyrido[3,4-b]indol.

Beispiel 27

2,7 g 2-Acetyl-1,5,7,9-tetramethyl-2,3,4,9-tetrahydro-1H-pyrido[3,4-b]indol werden analog Beispiel 13 mit 50 ml 1,5n-Natronlauge und 50 ml Aethanol 5 Stunden am Rückfluss gekocht. Es wird analog den Aufarbeitungsvorschriften von Beispiel 13 reines 1,5,7,9-Tetramethyl-2,3,4,9-tetrahydro-1H-pyrido[3,4-b]indol vom Smp. 86–87° erhalten.

Das benötigte Ausgangsmaterial kann wie folgt erhalten werden:

a) Analog Beispiel 13a wird aus 12,0 g 1,5,7-Trimethyl-2,3,4,9-tetrahydro-1H-pyrido[3,4-b]indol reines 2-Acetyl-1,5,7-trimethyl-2,3,4,9-tetrahydro-1H-pyrido-[3,4-b]indol erhalten.

b) Analog Beispiel 13c wird aus 11,5 g 2-Acetyl-1,5,7-trimethyl-2,3,4,9-tetrahydro-1H-pyrido[3,4-b]indol, reines 2-Acetyl-1,5,7,9-tetramethyl-2,3,4,9-tetrahydro-1H-pyrido[3,4-b]indol erhalten.

Beispiel 28

27,2 g 3,5-Dimethyl-phenylhydrazin werden mit 38,0 g N-Benzyl-3-piperidon in 200 ml abs. Alkohol 6 Stunden am Rückfluss gekocht. Das Gemisch wird zur Trockene eingedampft und der Rückstand aus Aether-Pentan kristallisiert, wobei das reine N-Benzyl-piperidinon-3-[N'-(3,5-dimethyl-phenyl)-hydrazon] erhalten wird. 30 g N-Benzyl-piperidinon-3-[N'-(3,5-dimethyl-phenyl)-hydrazon] werden in 750 ml Alkohol abs. gelöst und diese Lösung mit Salzsäuregas gesättigt. Nach 3 Stunden Kochen am Rückfluss wird das Gemisch zur Trockene eingedampft und der Rückstand in Wasser gelöst und mit Aether ausgeschüttelt. Die saure wässerige Phase wird anschliessend mit Natronlauge basisch gestellt und mit Methylenchlorid ausgeschüttelt. Die erhaltenen organischen Phasen werden mit Wasser gewaschen, und über Magnesiumsulfat getrocknet. Der erhaltene Rückstand wird an einer Säule mit 200 g basischem Silicagel (Elutionsmittel Toluol + 2% Methanol) chromatographiert. Es wird dabei nach Umkristallisation aus Aether-Petroläthan 2-Benzyl-5,7-dimethyl-2,3,4,9-tetrahydro-1H-pyrido[3,4-b]indol erhalten. Smp. 157 –159°.

Beispiel 29

2,9 g 2-Benzyl-5,7-dimethyl-2,3,4,9-tetrahydro-1H-pyrido [3,4-b]indol werden in 50 ml Tetrahydrofuran gelöst und unter Rühren bei -78° 6 ml Butyllithium in Hexan eingetropft. Das Gemisch wird 30 Minuten bei -78° gerührt und anschliessend bei gleichen Temperatur eine Lösung von 1,42 g Methyljodid in 10 ml Tetrahydrofuran im Verlaufe von 10 Minuten eingetropft. Das Gemisch wird langsam auf Raumtemperatur gebracht und 2 Stunden stehen gelassen. Anschliessend wird das Gemisch mit Eiswasser versetzt und am Vakuum zur Trockene eingedampft. Der Rückstand wird mit 2n-Natronlauge alkalisch gestellt und mit Wasser und Methylenchlorid ausgeschüttelt. Die organischen Phasen werden mit Wasser gewaschen, über Magnesiumsulfat getrocknet und das Lösungsmittel am Vakuum entfernt. Der Rückstand wird aus Aether-Pentan kristallisiert und ergibt reines 2-Benzyl-5,7,9-trimethyl-2,3,4,9-tetrahydro-1H-pyrido-[3,4-b]indol vom Smp. 135-136°.

Beispiel 30

3,04 g 2-Benzyl-5,7,9-trimethyl-2,3,4,9-tetrahydro-1H-pyrido[3,4-b]indol werden unter Zusatz von 1,0 g konz. Schwefelsäure in 100 ml abs. Alkohol gelöst und nach Zugabe von 1 g 5-%iger Palladiumkohle bei Raumtemperatur und 5 bar Wasserstoffdruck katalytisch hydriert. Nach Beendigung der Wasserstoffaufnahmes wird vom Katalysator abfiltriert und das Filtrat zu Trockene eingedampft. Der Rückstand wird in Wasser gelöst, mit 2n-Natronlauge alkalisch gestellt und mit Methylenchlorid ausgezogen. Die organische Phase wird mit Wasser gewaschen, über Magnesiumsulfat getrocknet und das Lösungsmittel am Vakuum entfernt. Der Rückstand wird

aus Aether-Pentan kristallisiert und ergibt reines 5,7,9-Trimethyl-2,3,4,9-tetrahydro-1H-pyrido[3,4-b]indol vom Smp. 142 -143°.

Patentansprüche

1. Diazatricyclen der Formel

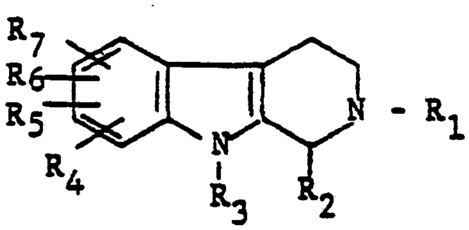

(I)

worin

$R_1$ Wasserstoff, Niederalkyl, Niederalkenyl, 2-Propinyl, Cycloalkylniederalkyl, Hydroxyniederalkyl oder gegebenenfalls substituiertes Benzyl,

$R_2$ Wasserstoff oder Niederalkyl,

$R_3$ Wasserstoff oder Niederalkyl bedeutet,

$R_4$ und $R_5$ Niederalkyl, und

$R_6$ und $R_7$ Wasserstoff, Niederalkyl, Niederalkoxy oder Halogen darstellen,

sowie Salze von solchen Verbindungen.

2. Verbindungen der im Anspruch 1 angegebenen Formel I, worin $R_1$ Wasserstoff, Niederalkyl oder Niederalkenyl mit bis zu 7 Kohlenstoffatomen, 2-Propinyl, Cycloalkylniederalkyl mit 4-10 Kohlenstoffatomen, Hydroxyniederalkyl mit 2 - 7 Kohlenstoffatomen, oder gegebenenfalls durch Niederalkyl oder Niederalkoxy mit bis zu 7 Kohlenstoffatomen oder durch Halogen substituiertes Benzyl, $R_2$ Wasserstoff oder Niederalkyl mit bis zu 7 Kohlenstoffatomen, $R_3$ Wasserstoff oder Niederalkyl mit bis zu 7 Kohlenstoffatomen bedeutet, $R_4$ und $R_5$ Niederalkyl mit bis zu 7 Kohlenstoffatomen, und $R_6$ und $R_7$ Wasserstoff, Niederalkyl oder Niederalkoxy mit bis zu 7 Kohlenstoffatomen oder Halogen darstellen.

3. Verbindungen der im Anspruch 1 angegebenen Formel I, worin $R_1$ Wasserstoff, Niederalkyl oder Niederalkenyl mit bis 4 Kohlenstoffatomen, 2-Propinol, Cycloalkylniederalkyl mit 4-8 Kohlenstoffatomen, Hydroxyniederalkyl mit 2-4 Kohlenstoffatomen, oder gegebenenfalls durch Niederalkyl oder Niederalkoxy mit bis zu 4 Kohlenstoffatomen oder durch Halogen substituiertes Benzyl, $R_2$ Wasserstoff oder Niederalkyl mit bis zu 4 Kohlenstoffatomen, $R_3$ Wasserstoff oder Niederalkyl mit bis zu 4 Kohlenstoffatomen bedeutet, $R_4$ und $R_5$ Niederalkyl mit bis zu 4 Kohlenstoffatomen und $R_6$ und $R_7$ Wasserstoff, Niederalkyl oder Niederalkoxy mit bis zu 4 Kohlenstoffatomen oder Halogen darstellen.

4. Verbindungen der im Anspruch 1 angegebenen Formel I, worin $R_1$ Wasserstoff oder Methyl, 2-Propinyl, 2-Hydroxyäthyl, Benzyl oder p-Fluorbenzyl, $R_2$ Wasserstoff oder Methyl, $R_3$ Wasserstoff oder Methyl, $R_4$ und $R_5$ Methyl, $R_6$ Methyl oder Wasserstoff und $R_7$ Wasserstoff darstellen.

5. 5,7,9-Trimethyl-2,3,4,9-tetrahydro-1H-pyrido [3,4-b]indol, sowie Salze dieser Verbindung.

6. 1,5,7,9-Tetramethyl-2,3,4,9-tetrahydro-1H-pyrido [3,4-b]indol, sowie Salze dieser Verbindung.

7. Die pharmazeutisch annehmbaren Säureadditionssalze der Verbindungen gemäss den Ansprüchen 1-6.

8. Verfahren zur Herstellung von Diazatricyclen der Formel

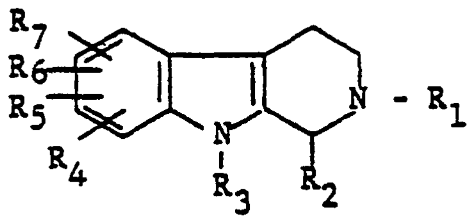 (I)

worin

$R_1$ Wasserstoff, Niederalkyl, Niederalkenyl, 2-Propinyl, Cycloalkylniederalkyl, Hydroxyniederalkyl oder gegebenenfalls substituiertes Benzyl,

$R_2$ Wasserstoff oder Niederalkyl,

$R_3$ Wasserstoff oder Niederalkyl bedeutet,

$R_4$ und $R_5$ Niederalkyl, und

$R_6$ und $R_7$ Wasserstoff, Niederalkyl, Niederalkoxy oder Halogen darstellen,

sowie Salze von solchen Verbindungen, dadurch gekennzeichnet, dass man

a) eine Verbindung der Formel

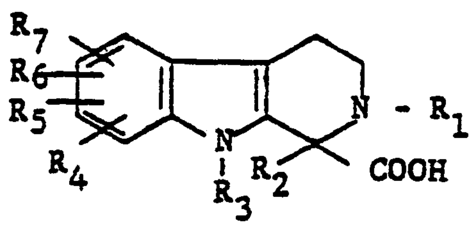 (II)

decarboxyliert, oder

b) eine Verbindung der Formel

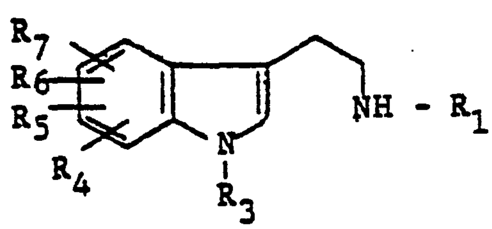 (IV)

mit einer Verbindung der Formel

$R_2$ - CHO (VI)

umsetzt, oder

c) in einer Verbindung der Formel

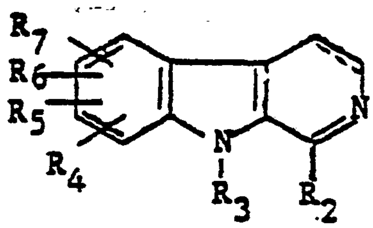

(VII)

oder

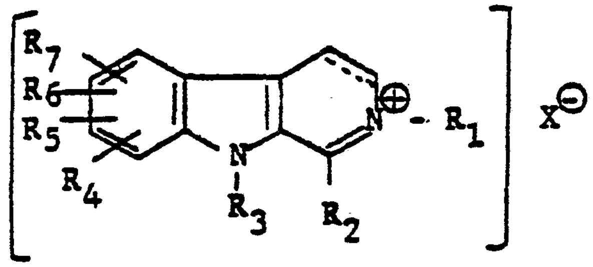

(VIII)

die zusätzlich eine Doppelbindung in 3,4-Stellung aufweisen kann, worin $X^{\ominus}$ ein Anion bedeutet, die C=N-Doppelbindung und eine gegebenenfalls vorhandene 3,4-Doppelbindung reduziert, oder

d) eine Verbindung der Formel

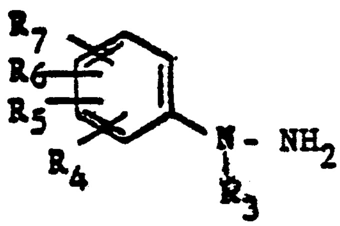

(XII)

mit einer Verbindung der Formel

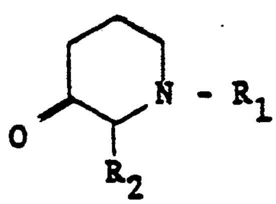

(XIII)

oder mit dem Hydrat oder einem Ketal derselben umsetzt, oder

e) in einer Verbindung der Formel

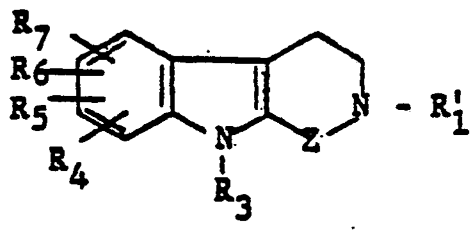

(XIV)

worin

Z die -CO- oder die $>CH-R_2$-Gruppe bedeutet und

$R_1'$ entweder $R_1$ oder die $-CO-R_1^a$-Gruppe darstellt,
wobei

$R_1^a$ einen um eine Methylengruppe verminderten, sonst aber der oben angegebenen Definition für $R_1$ entsprechenden Rest bedeutet,
wobei mindestens eines der Symbole Z und $R_1'$ eine Carbonylgruppe darstellt bzw. enthält,

vorhandene Carbonylgruppen zu Methylengruppen reduziert, oder

f) zur Herstellung der Verbindungen der Formel I, in denen $R_1$ und/oder $R_3$ Wasserstoff bedeutet, eine entsprechende Verbindung, in der an Stelle von $R_1$ und/oder $R_3$ eine durch Solvolyse oder durch Reduktion abspaltbare Schutzgruppe vorliegt, diese durch Solvolyse bzw. Reduktion entfernt, und, wenn erwünscht, eine verfahrensgemäss erhaltene Verbindung der Formel I in eine andere Verbindung der Formel I umwandelt, und/oder, wenn erwünscht, ein verfahrensgemäss erhaltenes Salz in die freie Verbindung oder in ein anderes Salz oder eine verfahrensgemäss erhaltene freie Verbindung in ein Salz umwandelt und/oder wenn erwünscht, ein verfahrensgemäss erhaltenes Isomerengemisch in die einzelnen Isomeren trennt.

9. Verfahren nach Anspruch 8, dadurch gekennzeichnet, dass man von einer auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht, oder einen Ausgangsstoff unter den Reaktionsbedingungen bildet, oder eine Reaktionskomponente gegebenenfalls in Form ihrer Derivate, wie eines Salzes verwendet.

10. Verfahren nach Anspruch 8, dadurch gekennzeichnet, dass man eine Verbindung gemäss Anspruch 4 oder ein Säureadditionssalz einer solchen herstellt.

11. Verfahren nach Anspruch 8, dadurch gekennzeichnet, dass man eine Verbindung gemäss Anspruch 5 oder ein Säureadditionssalz einer solchen herstellt.

12. Verfahren nach Anspruch 8, dadurch gekennzeichnet, dass man eine der in den Beispielen beschriebenen neuen Verbindungen oder ein Säureadditionssalz einer solchen herstellt.

13. Pharmazeutische Zusammensetzungen, gekennzeichnet durch einen Gehalt an einer Verbindung gemäss Anspruch 1 oder einem pharmazeutisch annehmbaren Säureadditionssalz derselben, und mindestens einen pharmazeutischen Trägerstoff.

14. Pharmazeutische Zusammensetzungen, gekennzeichnet durch einen Gehalt an einer Verbindung gemäss einem der Ansprüche 2 bis 6 oder einem pharmazeutisch annehmbaren Säureadditionssalz derselben, und mindestens einem pharmazeutischen Trägerstoff.

15. **Pharmazeutische Zusammensetzungen, gekennzeichnet durch einen Gehalt an 5,7,9-Trimethyl-2,3,4,9-tetrahydro-1H-pyrido[3,4-b]indol, sowie Salze dieser Verbindung oder einem pharmazeutisch annehmbaren Säureadditionssalz desselben, und mindestens einem pharmazeutischen Trägerstoff.**

16. Pharmazeutische Zusammensetzungen, gekennzeichnet durch einen Gehalt an 1,5,7,9-tetrahydro-1H-pyrido[3,4-b]indol, sowie Salze dieser Verbindung oder einem pharmazeutisch annehmbaren Säureadditionssalz desselben, und mindestens einem pharmazeutischen Trägerstoff.

17. Verwendung einer Verbindung gemäss einem der Ansprüche 1 bis 6 oder eines pharmazeutisch annehmbaren Salzes einer solchen als Antidepressivum.

18. Diazatricyclen nach Anspruch 1 zur Anwendung in einem Verfahren zur therapeutischen Behandlung des menschlichen oder tierischen Körpers.

19. Diazatricyclen nach einem der Ansprüche 2-7 zur Anwendung in einem Verfahren zur therapeutischen Behandlung des menschlichen und tierischen Körpers.

20. 5,7,9-Trimethyl-2,3,4,9-tetrahydro-1H-pyrido[3,4-b]indol zur Anwendung in einem Verfahren zur therapeutischen Behandlung des menschlichen und tierischen Körpers.

21. 1,5,7,9-Tetramethyl-2,3,4,9-tetrahydro-1H-pyrido-[3,4-b]indol zur Anwendung in einem Verfahren zur therapeutischen Behandlung des menschlichen und tierischen Körpers.

0017727

Nummer der Anmeldung

Europäisches Patentamt

# EUROPÄISCHER RECHERCHENBERICHT

EP 80 10 0821.0

| Kategorie | EINSCHLÄGIGE DOKUMENTE<br>Kennzeichnung des Dokuments mit Angabe, soweit erforderlich, der maßgeblichen Teile | betrifft Anspruch | KLASSIFIKATION DER ANMELDUNG (Int.Cl.³) |
|---|---|---|---|
| | US - A - 3 071 589 (STERLING DRUG)<br>* Ansprüche 1, 4 bis 6; Spalte 4, Zeilen 23 bis 28 *<br>-- | 1,8,13 | C 07 D 471/04<br>A 61 K 31/435<br>//(C 07 D 471/04, 221/00, 209/00) |
| | US - A - 3 455 943 (AMERICAN CYANAMID)<br>* Spalte 2, Zeilen 50 bis 58 *<br>-- | 13 | |
| | US - A - 3 718 657 (ABBOTT LABORATORIES)<br>* Zusammenfassung *<br>-- | 1,13 | RECHERCHIERTE SACHGEBIETE (Int. Cl.³) |
| | FR - M - 3 395M (ROUSSEL-UCLAF)<br>* Anspruch *<br>---- | 1,13 | A 61 K 31/435<br>C 07 D 471/04 |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 23-07-1980 | FROELICH |